(19) Europäisches Patentamt
European Patent Office
Office européen des brevets

(11) **EP 4 335 435 A1**

(12) **EUROPEAN PATENT APPLICATION**

(43) Date of publication:
**13.03.2024 Bulletin 2024/11**

(21) Application number: **23196274.7**

(22) Date of filing: **08.09.2023**

(51) International Patent Classification (IPC):
***A61K 9/16*** *(2006.01)*

(52) Cooperative Patent Classification (CPC):
**A61K 9/1647**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **09.09.2022 JP 2022143697**
 **14.10.2022 JP 2022165645**
 **20.04.2023 JP 2023069557**
 **16.05.2023 JP 2023081051**

(71) Applicant: **Ricoh Company, Ltd.**
**Tokyo 143-8555 (JP)**

(72) Inventors:
• **SATO, Yuichi**
 **Tokyo, 143-8555 (JP)**
• **INOUE, Ryota**
 **Tokyo, 143-8555 (JP)**
• **MORITANI, Tatsuru**
 **Tokyo, 143-8555 (JP)**

(74) Representative: **Marks & Clerk LLP**
 **15 Fetter Lane**
 **London EC4A 1BW (GB)**

(54) **SUSTAINED RELEASING PARTICLE AND METHOD OF PRODUCING SUSTAINED RELEASING PARTICLE**

(57) A sustained releasing particle contains a water-soluble physiologically active substance and a fat-soluble base material, wherein the sustained releasing particle has a ratio of a surface area to a volume is 0.6 or less.

**EP 4 335 435 A1**

**Description**

BACKGROUND

Technical Field

The present disclosure relates to a sustained releasing particle and a method of producing a sustained releasing particle.

Related Art

Sustained releasing products containing physiologically active substances such as functional foods and pharmaceuticals have been developed. The physiologically active substance in a sustained releasing product is gradually dissolved and absorbed into the human body.

The physiologically active substance includes a water-soluble physiologically active substance. As functional foods and pharmaceuticals, this water-soluble physiologically active substance is required to dissolve in the human body gradually. Using a fat-soluble base material is suitable to stably dissolve a physiologically active substance for a long period. However, in a particle of a fat-soluble material with a water-soluble physiologically active substance, the water-soluble physiologically active substance is likely to be unevenly distributed at the outer side in the particle. In such a particle, a large amount of the physiologically active substance elutes within a few hours after administration into the body, which is called an initial burst. A high amount of the physiologically active substance eluted during the initial burst causes adverse effects on the body due to the side effects or results in poor control over the administration cycle of the physiologically active substance. Therefore, the initial burst should be reduced.

In an attempt to reduce the initial burst, a microsphere has been proposed in WO2008/047863 that contains a physiologically active substance with the type and content regulated and a base material with the type, content, and properties regulated together with a controlled weight average particle size.

Also, a sustained releasing particle having a ratio of the surface area to the volume of 0.6 or less has been proposed in Unexamined Japanese Patent Application Publication No. 2021-147330.

SUMMARY

The present disclosure provides sustained releasing particles which prevents an initial burst and which have excellent sustained releasing properties suitable for functional foods and pharmaceuticals.

According to embodiments of the present disclosure, a sustained releasing particle is provided that contains a water-soluble physiologically active substance and a fat-soluble base material, wherein the sustained releasing particle has a ratio of a surface area to a volume is 0.6 or less.

As another aspect of embodiments of the present disclosure, a method of producing a sustained releasing particle is provided that includes discharging a liquid droplet containing a physiologically active substance, a base material, and a solvent and removing the solvent from the liquid droplet by a dry gas stream on condition that a difference ($T_d$ - $T_m$) between the dew point temperature ($T_d$) of the dry gas stream and the wet bulb temperature ($T_m$) of the liquid droplet discharged is 20 degrees Celsius or less.

BRIEF DESCRIPTION OF THE DRAWINGS

A more complete appreciation of embodiments of the present disclosure and many of the attendant advantages and features thereof can be readily obtained and understood from the following detailed description with reference to the accompanying drawings, wherein:

FIG. 1 illustrates an exemplary particle size distribution of particles produced in the method according to the present embodiment and particles produced by a spray drying method;
FIG. 2 is a schematic cross-sectional view of an example of a liquid-column-resonant liquid droplet discharger;
FIG. 3 is a schematic diagram illustrating an example of a particle production apparatus;
FIG. 4 is a schematic cross-sectional view of an example of a liquid droplet discharger used in the particle production apparatus;
FIG. 5 is a schematic cross-sectional view of another example of the liquid droplet discharger used in the particle production apparatus; and
FIG. 6 is a schematic cross-sectional view of an example of a Rayleigh fission liquid droplet discharger.

The accompanying drawings are intended to depict embodiments of the present disclosure and should not be interpreted to limit the scope thereof. The accompanying drawings are not to be considered as drawn to scale unless explicitly noted. Also, identical or similar reference numerals designate identical or similar components throughout the several views.

DETAILED DESCRIPTION

In describing embodiments illustrated in the drawings, specific terminology is employed for the sake of clarity. However, the disclosure of this specification is not intended to be limited to the specific terminology so selected and it is to be understood that each specific element includes all technical equivalents that have a similar function, operate in a similar manner, and achieve a similar result.

Referring now to the drawings, embodiments of the present disclosure are described below. As used herein, the singular forms "a," "an," and "the" are intended to include the plural forms as well, unless the context clearly indicates otherwise.

Sustained Releasing Particle

The sustained releasing particle of the present disclosure contains a water-soluble physiologically active substance and a fat-soluble base material, and any other optional materials.

The abovementioned sustained releasing particle has a ratio of the surface area to the volume of the sustained releasing particle (hereinafter also referred to as surface area/volume ratio) of 0.6 or less.

The inventors of the present invention have examined sustained releasing particles and acquired the following knowledge.

In the conventional technologies, the properties of particles have been determined based on such indices such as volume average particle size and weight average particle size, and surface area of particles. The inventors of the present invention have found that the sustained releasing particle using a fat-soluble base material and having a surface area/volume ratio of 0.6 or less can reduce the initial burst of a water-soluble physiologically active substance and have excellent sustained releasing properties.

The inventors of the present invention have also found that the initial burst of particles is related not only to the size and surface area of the particle, but also to the dew point temperature ($T_d$) of a dry gas stream when the solvent is removed from the liquid droplet and the wet bulb temperature ($T_m$) of the discharged (sometimes referred to as sprayed) liquid droplet.

The sustained releasing particle of the present disclosure demonstrates sustained releasing, preventing an initial burst.

The sustained releasing particle can be suitably produced by the method of producing the sustained releasing particle of the present disclosure, by which particles that demonstrate sustained releasing can be produced and an initial burst can be reduced, as described below.

In the present disclosure, the "particle" is also referred to as "microcapsule" or "microparticle".

The particle of the present disclosure includes at least one type of base material and at least one type of physiologically active substance having physiological activity, and any other optional materials.

In the present disclosure, "base material" refers to a component in the particle. It is a base that constitutes each particle.

The physiologically active substance has some physiological activity in vivo. In a preferable aspect, the physiological activity of the physiologically active substance changes upon chemical or physical stimuli such as heating, cooling, shaking, agitation, and pH change.

The "particle" in the present disclosure means a group of granular compositions each containing a base material and a physiologically active substance, unless otherwise stated. The particle of the present disclosure, a sustained releasing particle, continues releasing a physiologically active substance over a long period of time.

The "physiologically active substance" in the present disclosure is an effective component used to achieve physiological effects on living bodies. Examples thereof include, but are not limited to, low molecular weight compounds containing pharmaceutical compounds, food compounds, and cosmetic compounds, and high molecular weight compounds containing biological polymers such as proteins (e.g., antibodies, enzymes) and nucleic acids (e.g., DNA, RNA). The physiologically active substance demonstrates "physiological effects" at a target site. Such effects include, but are not limited to, quantitative and/or qualitative changes or impacts on living bodies, tissues, cells, proteins, DNA, and RNA. The "physiological activity" refers to the ability of a physiologically active substance to give a change or impact on a target site (e.g., target tissue). Preferably, the target site is a receptor present on or in a cell. In this case, the physiologically active substance binds to a specific receptor by physiological activity, transmitting an intracellular signal and demonstrating physiological effects.

The physiologically active substance includes a substance that is converted into a mature form by an enzyme in vivo and then binds to a specific receptor, thus demonstrating a physiological effect.

The physiologically active substance can be produced from an organism (human being or a non-human organism) or synthesized.

The particle of the present disclosure has physical properties such as the ratio of the surface area to the volume of the particle, particle size distribution, and particle size. The dew point temperature ($T_d$) of the dry gas stream when the solvent is removed from the liquid droplet and the wet bulb temperature ($T_m$) of the ejected liquid droplet is explained in the section on the method of producing a sustained releasing particle below.

Ratio of Surface Area to Volume-

The ratio of surface area to volume of the sustained releasing particle of the present disclosure is 0.6 or less, preferably 0.5 or less, and more preferably 0.3. The sustained releasing particle having a surface area/volume ratio of less than 0.6 is not a minute particle and can prevent initial burst.

Surface area/volume ratio can be calculated, for example, by using a concentrated system analyzer based on the dynamic light scattering ("FPAR-1000", available from Otsuka Electronics Co., Ltd.) or by using the surface area and volume obtained based on the number average particle size Dn obtained, for example, a particle size distribution analyzer by a laser diffraction scattering method ("LA-960", available from HORIBA, Ltd.), as follows.

The surface area/volume ratio is calculated using the following formula:

$$\text{Surface area/volume ratio} = (\text{sum of surface areas of each number average particle size Dn})/(\text{sum of volumes of each number average particle size Dn})$$

The sum of surface areas of each number average particle size Dn is calculated using the following formula:

$$\text{Sum of surface areas of each number average particle size Dn} = \text{Sum of "surface area} \times \text{existence ratio corresponding to each number average particle size Dn}$$

The sum of the volumes of each number average particle size Dn is calculated using the following formula:

$$\text{Sum of volumes of each number average particle size Dn} = \text{Sum of "volume} \times \text{abundance ratio corresponding to each number average particle size Dn}$$

A total of 0.1 g of DRIWEL K (Drywell, available from FUJIFILM Corporation) and 5.0 g of water are added to 0.003 g of the measured particles, and the resulting mixture is subjected to ultrasonic dispersion for 3 minutes, and thus obtained dispersion is used as a sample for measurement in the above apparatus.

[Measurement Conditions]

Transmittance (R): 85-95%.
Transmittance (B): 70-90%.
Algorithm Option: Standard Mode
Particle Size Distribution

Preferably, the particles of the present disclosure intrinsically have a narrow particle size distribution. Specifically, examples of indicators of the narrowness of the particle size distribution include, but not limited to, the relative span factor (R.S.F.) and the volume average particle size (Dv)/number average particle size (Dn). For example, the relative span factor (R.S.F.) is preferably in the range 0 < (R.S.F.) $\leq$ 1.2 and the volume average particle size (Dv)/number of particles (Dn) is between 1.00 and 1.50, both inclusive. The particle size distribution within the above range reduces the proportion of coarse particles compared to the desired particle size. This distribution allows easy and efficient filtration sterilization without clogging the sterilization filter, even when the particles are subjected to filtration sterilization before use like when the particles should be contained in a pharmaceutical composition. Such uniform-sized particles also result in the same amount of physiologically active substance and base material and the same surface area. This uniformity ensures that each particle elutes the same amount of the physiologically active substance so that the sustained releasing of the physiologically active substance can be highly controlled. In addition, such uniform particles can reduce production of small physiologically active substance particles not contained in the base material, resulting in particles with sustained releasing properties and less occurrence of initial burst.

Relative Span Factor (R.S.F.)

In the present disclosure, "relative span factor (R.S.F.)" is defined as (D90-D10)/D50. D90 represents the cumulative 90th percentile from the small particle side of the cumulative particle size distribution, D50 represents the cumulative 50th percentile from the small particle side of the cumulative particle size distribution, and D10 represents the cumulative 10th percentile from the small particle side of the cumulative particle size distribution. (R.S.F.) is preferably in the range 0 < (R.S.F.) $\leq$ 1.2, more preferably in the range 0 < (R.S.F.) $\leq$ 1.0, and even more preferably in the range 0 < (R.S.F.) $\leq$ 0.6.
(R.S.F.) can be measured, for example, using a concentrated system analyzer based on the dynamic light scattering ("FPAR-1000", available from Otsuka Electronics Co., Ltd.) or a particle size distribution analyzer by a laser diffraction scattering method ("LA-960", available from HORIBA, Ltd.).

Volume Average Particle Size (Dv)/Number Average Particle Size (Dn)

The ratio of the volume average particle size (Dv) to the number average particle size (Dn) is obtained by dividing the (Dv) by the number average particle size (Dn). The volume average particle size (Dv)/number average particle size (Dn) is preferably between 1.00 and 1.50, both inclusive, and more preferably between 1.00 and 1.20, both inclusive.
The volume average particle size (Dv) and the number average particle size (Dn) are measured, for example, by using a laser diffraction scattering particle size distribution analyzer (device name: Microtrac MT3000II, available from Microtrac Bell Corporation) or a particle size distribution analyzer by a laser diffraction scattering method ("LA-960", available from HORIBA, Ltd.).

Particle Size

The number average particle size (Dn) of the particles is preferably between 1 $\mu$m and 50 $\mu$m, both inclusive, and more preferably between 10 $\mu$m and 30 $\mu$m, both inclusive, but may be appropriately selected according to the purpose.
When the number average particle size (Dn) is between 1 $\mu$m and 50 $\mu$m, both inclusive, a sufficient amount of physiologically active substance can be retained, and the particles that can, for example, perform sustained releasing of the physiologically active substance for a long period of time can be produced. The volume average particle size (Dv) is more preferably between 1 $\mu$m and 50 $\mu$m, both inclusive, and even more preferably between 1 $\mu$m and 50 $\mu$m, both inclusive.
The number average particle size (Dn) of particles can be measured, for example, using a concentrated system analyzer based on the dynamic light scattering ("FPAR-1000", available from Otsuka Electronics Co., Ltd.), or a laser diffraction scattering particle size distribution analyzer (device name: Microtrac MT3000II, available from Microtrac Bell Corporation) or a particle size distribution analyzer by a laser diffraction scattering method ("LA-960", available from HORIBA, Ltd.).
Next, embodiments of particles will be described.
Generally, embodiments of the particles each containing the base material and the physiologically active substance include capsule particles in which the physiologically active substance is encapsulated in the base material, carrier particles in which the physiologically active substance is carried on the surface of the base material, and particles of other embodiments.
Capsule particles include dispersion capsule particles, in which the physiologically active substance is dispersed and encapsulated in the base material, and maldistribution capsule particles, in which the physiologically active substance is unevenly distributed and encapsulated in the base material. The forms of "encapsulation" used for capsule particles is not particularly limited as long as the physiologically active substance is temporarily or continuously retained in the base material.
Any dispersion capsule particles can be used as long as the physiologically active substance is dispersed and encapsulated in the base material. The physiologically active substance can be uniformly or non-uniformly dispersed in the base material.

In the maldistribution capsule particle, the physiologically active substance is unevenly distributed and included in the base material. In other words, the base material encapsulates the physiologically active substance in such a manner that the base material and the physiologically active substance are substantially separated in a particle.

An embodiment of the maldistribution capsule particle is a particle having a core containing the physiologically active substance and an exterior containing the base material enclosing the core. Examples of the maldistribution capsule particles include, but are not limited to, liposomes, micelles, and coated particles.

In the case where each particle contains base materials one of which is locally distributed in a certain area of the particle, the physiologically active substance may be differently dispersed depending on the type of the base material in which the physiologically active substance is encapsulated.

Examples of the dispersion capsule particles include, but are not limited to, the particles of the present disclosure, particles produced by the emulsion solvent diffusion method (ESD method), and particles produced by the spray drying method.

A particle according to the present disclosure may contain two or more types of base materials, and if two or more types of base materials are contained, one type of the base materials may be distributed more towards the surface of the particle. In this case, the physiologically active substance can be dispersed and encapsulated, to varying degrees, in both the base material distributed more toward the surface side of the particle (hereinafter also referred to as "surface base material") and the base material other than the surface base material (hereinafter also referred to as "internal base material").

Examples of this embodiment include an embodiment in which the physiologically active substance is distributed more toward the surface base material and an embodiment in which the physiologically active substance is distributed more toward the internal base material. However, an embodiment in which the physiologically active substance is distributed more toward the internal base material is preferred. The physiologically active substance is distributed more toward the internal base material, which enables preparation of sustained releasing particles in which the elution rate of the physiologically active substance is retarded.

The confirmation method of confirming that the particles are such an embodiment that contains at least two types of base materials, and that one of these at least two types of base materials is distributed more toward the surface side of the particles is not particularly limited, and may be appropriately selected according to the purpose.

One example of the confirmation method is to observe the particle cross section with a scanning electron microscope, a transmission electron microscope, or a scanning probe microscope.

Another example of the confirmation method is to measure the component of the surface base material using time-of-flight secondary ion mass spectrometry, and if the component is determined to be different from the component of the internal base material, the particles are confirmed to be the abovementioned embodiment.

Base Material

The base material is a material constituting particles. Therefore, the base material is preferably solid at room temperature.

The base material is not particularly limited as long as not being a substance that adversely affects the physiologically active substance contained as well, and may be a low molecular weight substance or a high molecular weight substance, but since the particles according to the present disclosure are preferably particles applied to living bodies, the base material is preferably a substance that is not toxic to living bodies.

The low molecular weight substance is preferably a compound with a weight average molecular weight of less than 15,000.

The high molecular weight substance is preferably a compound with a weight average molecular weight of 15,000 or more.

As mentioned above, only one or two or more base materials may be used, or a combination of any of the base materials described below. The base material used in the present disclosure preferably contains a biodegradable resin, preferably at least one of polylactic acid or lactic acid-glycolic acid copolymer.

The base material in the sustained releasing particles according to the present disclosure is fat-soluble. On the other hand, the base material in the production method of the sustained releasing particles according to the present disclosure described below is preferably, but not limited to, a fat-soluble one. In order to avoid duplicate explanations, fat-insoluble base materials will also be explained below.

A fat-soluble substance means a substance whose water/octanol partition coefficient (logP value) is 3 or more, which can be measured in accordance with JIS Z 7260-107.

Low Molecular Weight Substance

The low molecular weight substance which is fat-soluble is not particularly limited, and may be appropriately selected according to the purpose. An example of the low molecular weight substance which is fat-soluble is lipids.

The low molecular weight substance which is fat-insoluble is not particularly limited, and may be appropriately selected according to the purpose. Examples thereof include saccharides, cyclodextrins, amino acids, and organic acids.

Each of these may be used alone or in combination with others.

Lipids

The lipids may be appropriately selected according to the purpose, and examples of the lipids include, but are not limited to, middle-chain or long-chain monoglyceride, middle-chain or long-chain diglyceride, and middle-chain or long-chain triglyceride, phospholipid, plant oils (e.g., soybean oil, avocado oil, squalene oil, sesame oil, olive oil, corn oil, rapeseed oil, safflower oil, and sunflower oil), fish oil, seasoning oil, water-insoluble vitamin, fatty acid, and mixtures and derivatives thereof. Each of these can be used alone or in combination with others.

Saccharides

The saccharides may be appropriately selected according to the purpose, and examples of the saccharides include, but are not limited to, glucose, mannose, idose, galactose, fucose, ribose, xylose, lactose, sucrose, maltose, trehalose, turanose, raffinose, maltotriose, acarbose, cyclodextrins, amylose (starch), cellulose, and other monosaccharides and polysaccharides, and sugar alcohols (polyols) such as glycerin, sorbitol, lactitol, maltitol, mannitol, xylitol, and erythritol, and derivatives thereof. Each of these can be used alone or in combination with others.

Cyclodextrins

The cyclodextrin is not particularly limited, and may be appropriately selected according to the purpose. Examples thereof include hydroxypropyl-β-cyclodextrin, β-cyclodextrin, γ-cyclodextrin, α-cyclodextrin, and cyclodextrin derivative. Each of these can be used alone or in combination with others.

Amino Acids

The amino acids are not particularly limited, and may be appropriately selected according to the purpose. Examples thereof include valine, lysine, leucine, threonine, isoleucine, asparagine, glutamine, phenylalanine, aspartic acid, serine, glutamic acid, methionine, arginine, glycine, alanine, tyrosine, proline, histidine, cysteine, tryptophan, and derivatives thereof. Each of these can be used alone or in combination with others.

Organic Acids

The organic acids are not particularly limited, and may be appropriately selected according to the purpose. Examples thereof include adipic acid, ascorbic acid, citric acid, fumaric acid, gallic acid, glutaric acid, lactic acid, malic acid, maleic acid, succinic acid, tartaric acid, and derivatives thereof. Each of these can be used alone or in combination with others.

High Molecular Weight Substance

The high molecular weight substance which is fat-soluble is not particularly limited and may be appropriately selected according to the purpose. Examples thereof include poly(meth)acrylamide, poly(meth)acrylic acid, poly(meth)acrylic esters, polyarylamine, polyvinylpyrrolidone, polyvinyl acetate, biodegradable resin, polyglycolic acid, polyamino acids, gelatin, fibrin, and other proteins.

The high molecular weight substance which is not fat-soluble is not particularly limited and may be appropriately selected according to the purpose. Examples thereof include water-soluble cellulose, polyalkylene glycol, polyvinyl alcohol, polysaccharides, and derivatives thereof.

Each of these can be used alone or in combination with others.

Poly(Meth)Acrylamide

The poly(meth)acrylamide is not particularly limited and may be appropriately selected according to the purpose.

Examples thereof include polymers of monomers such as N-methyl(meth)acrylamide, N-ethyl(meth)acrylamide, N-propyl(meth)acrylamide, N-butyl(meth)acrylamide, N-benzyl(meth)acrylamide, N-hydroxyethyl(meth)acrylamide, N-phenyl(meth)acrylamide, N-tolyl(meth)acrylamide, N-(hydroxyphenyl)(meth)acrylamide, N-(sulfamoylphenyl)(meth)acrylamide, N-(phenylsulfonyl)(meth)acrylamide, N-(tolylsulfonyl)(meth)acrylamide, N,N-dimethyl(meth)acrylamide, N-methyl-N-phenyl(meth)acrylamide, and N-hydroxyethyl-N-methyl(meth)acrylamide. Any of the above-listed monomers may be polymerized alone, or a combination thereof may be polymerized together. Moreover, the above-listed polymers may be used alone or in combination.

Poly(Meth)Acrylic Acid

The poly(meth)acrylic acid is not particularly limited, and may be appropriately selected according to the purpose. Examples thereof include a homopolymer such as polyacrylic acid and polymethacrylic acid, and a copolymer such as an acrylic acid-methacrylic acid copolymer. Each of these can be used alone or in combination with others.

Poly(Meth)Acrylic Esters

The poly(meth)acrylic esters are not particularly limited, and may be appropriately selected according to the purpose. Examples thereof include polymers of monomers such as ethylene glycol di(meth)acrylate, diethylene glycol di(meth)acrylate, propylene glycol di(meth)acrylate, glycerol poly(meth)acrylate, polyethylene glycol (meth)acrylate, trimethylolpropane tri(meth)acrylate, pentaerythritol tetra(meth)acrylate, and 1,3-butyleneglycol di(meth)acrylate. Any of the above-listed monomers may be polymerized alone, or a combination thereof may be polymerized together. Moreover, the above-listed polymers may be used alone or in combination.

Polyarylamine

The polyallylamine is not particularly limited and may be appropriately selected according to the purpose. Examples thereof include diallylamine, and triallylamine. Each of these can be used alone or in combination with others.

Polyvinylpyrrolidone

As the polyvinylpyrrolidone, a commercial product may be used. The commercial product of the polyvinylpyrrolidone is not particularly limited and may be appropriately selected according to the purpose. Examples thereof include PLASDONE C-15 (available from ISP TECHNOLOGIES); KOLLIDON VA 64, KOLLIDON K-30, and KOLLIDON CL-M (all available from KAWARLAL); and KOLLICOAT IR (available from BASF). Each of these can be used alone or in combination with others.

Polyvinyl Acetate

The polyvinyl acetate is not particularly limited and may be appropriately selected according to the purpose. Examples thereof include vinyl acetate-crotonic acid copolymer, and vinyl acetate-itaconic acid copolymer. Each of these can be used alone or in combination with others.

Biodegradable Resin

The biodegradable resin is not particularly limited and may be appropriately selected according to the purpose. Examples thereof include biodegradable polyester. Examples of the biodegradable polyester include: polylactic acid; poly-$\varepsilon$-caprolactone; succinate-based polymers (lactic acid-glycolic acid copolymers), such as polyethylene succinate, polybutylene succinate, and polybutylene succinate adipate; polyhydroxyalkanoate, such as polyhydroxypropionate, polyhydroxy butyrate, and polyhydroxyparylate; and polyglycolic acid. Each of these can be used alone or in combination with others. Among the above-listed examples, polylactic acid is preferable because the polylactic acid has high biocompatibility, and can gradually elute a physiologically active substance contained therein.

Polylactic Acid

The weight average molecular weight of the polylactic acid is not particularly limited and may be appropriately selected according to the purpose. The weight average molecular weight of the polylactic acid is preferably 5,000 or more but 100,000 or less, more preferably 10,000 or more but 70,000 or less, further preferably 10,000 or more but 50,000 or less, even more preferably 40,000 or more, and particularly preferably 40,000 or more but 50,000 or less.

The content of the polylactic acid is not particularly limited and may be appropriately selected according to the purpose, but is preferably 50% by mass or more, more preferably 50% by mass or more but 99% by mass or less, further preferably 75% by mass or more but 99% by mass or less, and particularly preferably 80% by mass or more but 99% by mass or less, relative to a mass of the base material.

Polyglycolic Acid

The polyglycolic acid is not particularly limited and may be appropriately selected according to the purpose. Examples thereof include: a lactic acid-glycolic acid copolymer that is a copolymer including a structural unit derived from lactic acid and a structural unit derived from glycolic acid; a glycolic acid-caprolactone copolymer that is a copolymer including a structural unit derived from glycolic acid and a structural unit derived from caprolactone; and a glycolic acid-trimethylene carbonate copolymer that is a copolymer including a structural unit derived from glycolic acid and a structural unit derived from trimethylene carbonate. Each of these can be used alone or in combination with others. Among the above-listed examples, a lactic acid-glycolic acid copolymer (hereinafter sometimes referred to as "polylactic acid-glycolic acid copolymer" or "polylactic acid-glycolic acid") is preferable because of its high biocompatibility, can gradually elute a physiologically active substance contained therein, and can store the physiologically active substance contained therein over a long period of time.

The lactic acid-glycolic acid copolymer may be PURASORB PDLG5004, PURASORB PDLG5010, PURASORB PDLG7510, PURASORB PDLG7507, PURASORB PDLG5002A, PURASORB PDLG5002, PURASORB PDLG7502A (available from Corbion), RG502, RG502H, RG503, RG503H, RG504, RG504H (available from Sigma-Aldrich).

Preferably, the weight average molecular weight of the lactic acid-glycolic acid copolymer is not particularly limited and may be appropriately selected according to the purpose. The weight average molecular weight of the lactic acid-glycolic acid copolymer is preferably 2,000 or more but 250,000 or less, more preferably 2,000 or more but 100,000 or less, further preferably 40,000 or more, and particularly preferably 40,000 or more but 50,000 or less.

In the lactic acid-glycolic acid copolymer, the molar ratio (L:G) between a structural unit (L) derived from lactic acid and another structural unit (G) derived from glycolic acid is not particularly limited and may be appropriately selected according to the purpose, but is preferably from 1:99 to 99:1, more preferably from 25:75 to 99:1, further preferably from 30:70 to 90:10, and particularly preferably from 50:50 to 85:15.

The content of the lactic acid-glycolic acid copolymer is not particularly limited and may be appropriately selected according to the purpose, but is preferably 50% by mass or more, more preferably 50% by mass or more but 99% by mass or less, further preferably 75% by mass or more but 99% by mass or less, and particularly preferably 80% by mass or more but 99% by mass or less, relative to a mass of the base material.

Polyamino Acid

The polyamino acid is not particularly limited and may be appropriately selected according to the purpose. The polyamino acid may be a polymer of any selected combination of the above-listed amino acids, but preferably a polymer of single amino acid. Preferable Examples of the polyamino acid include: homopolymers of amino acid, such as poly-α-glutamic acid, poly-γ-glutamic acid, polyaspartic acid, polylysine, polyarginine, polyornithine, and poly serine; and copolymers thereof. Each of these can be used alone or in combination with others.

Gelatin

The gelatin is not particularly limited and may be appropriately selected according to the purpose. Examples of the gelatin include alkali processed gelatin, acid processed gelatin, gelatin hydrolysate, enzymatically dispersed gelatin, and derivatives thereof. Each of these can be used alone or in combination with others.

A natural dispersant polymer used for the gelatin derivative is not particularly limited and may be appropriately selected according to the purpose. Examples thereof include protein, polysaccharides, and nucleic acid. Examples thereof also include a copolymer including a natural dispersant polymer or a synthetic dispersant polymer. Each of these can be used alone or in combination with others.

The gelatin derivative refers to gelatin derivatized by covalently binding a hydrophobic group to a gelatin molecule.

The hydrophobic group is not particularly limited and may be appropriately selected according to the purpose. Examples of the hydrophobic group include: polyesters, such as polylactic acid, polyglycolic acid, and poly-ε-caprolactone; lipids, such as cholesterol and phosphatidyl ethanolamine; aromatic groups including alkyl groups and benzene rings; heteroaromatic groups; and mixtures thereof.

Protein

The protein is not particularly limited and may be appropriately selected according to the purpose, as long as the protein for use does not adversely affect the physiological activity of the physiologically active substance. Examples of the protein include collagen, fibrin, and albumin. Each of these can be used alone or in combination with others.

Water-soluble Cellulose

The water-soluble cellulose is not particularly limited and may be appropriately selected according to the purpose. Examples of the water-soluble cellulose include: alkyl cellulose, such as methyl cellulose, and ethyl cellulose; hydroxyalkyl cellulose, such as hydroxyethyl cellulose and hydroxypropyl cellulose; and hydroxyalkyl alkyl cellulose, such as hydroxyethyl methyl cellulose and hydroxypropyl methyl cellulose. Each of these can be used alone or in combination with others. Among the above-listed examples, hydroxypropyl cellulose and hydroxypropyl methyl cellulose are preferable, and hydroxypropyl cellulose is more preferable because of high biocompatibility and high solubility to a solvent for producing particles.

Hydroxypropyl Cellulose

As the hydroxypropyl cellulose, various products having different viscosities are available on the market from manufacturers. Any of such commercial products can be used for the base material according to the present disclosure. A viscosity of a 2% by mass hydroxypropyl cellulose aqueous solution (20 degrees Celsius) is not particularly limited and may be appropriately selected according to the purpose, but is preferably 2.0 mPa·s (centipoise, cps) or more but 4,000 mPa·s (centipoise, cps) or less.
Moreover, it seems that the viscosity of the hydroxypropyl cellulose depends on a weight average molecular weight, substitution degree, and molecular weight of the hydroxypropyl cellulose.
The weight average molecular weight of the hydroxypropyl cellulose is not particularly limited and may be appropriately selected according to the purpose, but is preferably 15,000 or more but 400,000 or less. The weight average molecular weight can be measured by gel permeation chromatography (GPC).
A commercial product of the hydroxypropyl cellulose is not particularly limited and may be appropriately selected according to the purpose. Examples of the commercial product of the hydroxypropyl cellulose include HPC-SSL having a molecular weight of 15,000 or more but 30,000 or less and viscosity of 2.0 mPa·s or more but 2.9 mPa·s or less, HPC-SL having a molecular weight of 30,000 or more but 50,000 or less and viscosity of 3.0 mPa·s or more but 5.9 mPa·s or less, HPC-L having a molecular weight of 55,000 or more but 70,000 or less and viscosity of 6.0 mPa s or more but 10.0 mPa s or less, HPC-M having a molecular weight of 110,000 or more but 150,000 or less and viscosity of 150 mPa·s or more but 400 mPa·s or less, and HPC-H having a molecular weight of 250,000 or more but 400,000 or less and viscosity of 1,000 mPa s or more but 4,000 mPa s or less (all available from Nippon Soda Co., Ltd.). Each of these can be used alone or in combination with others. Among the above-listed examples, HPC-SSL having a molecular weight of 15,000 or more but 30,000 or less and viscosity of 2.0 mPa·s or more but 2.9 mPa·s or less is preferable. The molecular weights of the above-listed commercial products can be measured by gel permeation chromatography (GPC), and the viscosity thereof is measured using a 2% by mass aqueous solution (20 degrees Celsius).

Polyalkylene Glycol

The polyalkylene glycol is not particularly limited and may be appropriately selected according to the purpose. Examples thereof include polyethylene glycol (PEG), polypropylene glycol, polybutylene glycol, and copolymers thereof. Each of these can be used alone or in combination with others.

Polyvinyl Alcohol

The polyvinyl alcohol is not particularly limited and may be appropriately selected according to the purpose. Examples of the polyvinyl alcohol include silanol-modified polyvinyl alcohol, carboxyl-modified polyvinyl alcohol, and acetoacetyl-modified polyvinyl alcohol. Each of these can be used alone or in combination with others.

Polysaccharides

The polysaccharides are not particularly limited and may be appropriately selected according to the purpose. Examples of the polysaccharides include chitin, chitosan, hyaluronic acid, alginic acid, starches, and pectin. Each of these can be used alone or in combination with others.

Since the base material is preferably a material with which resulting particles including the material can be added to pharmaceutical formulations, functional foods, and functional cosmetics, the base material is preferably a material that does not have biological toxicity, particularly, a biodegradable material, such as a biodegradable polymer.

A content of the base material is preferably 50% by mass or more but 99.99% by mass or less, and more preferably 70% by mass or more but 99% by mass or less, relative to the total amount of the particles. When the content of the base material is 50% by mass or more but 99.99% by mass or less, relative to the total amount of the particles, the physiologically active substance can be encapsulated in the base material, which helps gradually release the physiologically active substance.

Physiologically Active Substance

Physiologically active substances are active ingredients used to exert physiological effects on living bodies. Examples of physiologically active substances include physiologically active substances contained in pharmaceutical compositions, physiologically active substances contained in functional foods, and physiologically active substances contained in functional cosmetics. Each of these can be used alone or in combination with others.

The physiologically active substance in the sustained releasing particles according to the present disclosure is water-soluble. On the other hand, the physiologically active substance in the production method of the sustained releasing particles according to the preset disclosure described below is preferably water-soluble, but is not limited to water-soluble substances. In order to avoid redundant explanations, physiologically active substances other than water-soluble ones will be included below.

Water-soluble substances are substances with a water/octanol partition coefficient (logP value) of less than 3, which can be measured in accordance with JIS Z 7260-107.

Poorly water-soluble substances mean substances with a water/octanol partition coefficient (logP value) of 3 or more, which can be measured in accordance with JIS Z 7260-107.

Physiologically Active Substances Contained in Pharmaceutical Compositions

The physiologically active substances contained in pharmaceutical compositions are not particularly limited, and may be appropriately selected according to the purpose. Examples include nucleic acids, polypeptides including proteins, saccharides, lipids, and low molecular weight compounds. Each of these can be used alone or in combination with others.

Nucleic Acid

Examples of the nucleic acid typically include DNA, RNA, and a combination thereof, which may be substituted with chemically-modified nucleic acid, the sequence of which is either partially or entirely chemically modified. Moreover, examples of the nucleic acid also include chemically synthetized nucleic acid analogues, such as peptide nucleic acid (PNA) and morpholino antisense oligo.

When the aim is to prevent expression of a target gene, examples of the nucleic acid include an antisense nucleic acid for a transcription product of the target gene or part of the transcription product, a nucleic acid having ribozyme activity that causes specific cleavage of the transcription product of the target gene, a short-chain nucleic acid having a function of preventing expression of the target gene by RNAi interference, microRNA (miRNA), aptamer, locked nucleic acid obtained by modifying oligonucleotide.

Polypeptide

A polypeptide is a polymer formed of a plurality of amino acids. Among polypeptides, those having a higher-order structure and exhibiting a function derived from the higher-order structure are particularly referred to as proteins.

For example, the polypeptide includes both a polypeptide that is not modified from the original state exiting in the nature, and a polypeptide that is modified.

Examples of the modification include acetylation, acylation, ADP-ribosylation, amidation, a covalent bond of flavin, a covalent bond of a heme moiety, a covalent bond of nucleotide or a nucleotide derivative, a covalent bond of lipid or a lipid derivative, a covalent bond of phosphatidylinositol, crosslink, cyclization, formation of a disulfide bond, demethylation, formation of covalent crosslink, formation of cystine, formation of pyroglutamate, formylation, γ-carboxylation, glycosylation, formation of GPI anchor, hydroxylation, iodination, methylation, myristoylation, oxidation, a protein decomposition treatment, phosphorylation, prenylation, racemization, selenoylation, sulfation, tRNA mediation addition of amino acid to protein, such as arginylation, and ubiquitination.

When the aim is to inhibit or prevent a function of a target protein, examples of the protein include a target protein variant that is dominant-negative to the target protein, and antibody that binds to the target protein.

The antibody may be a polyclonal antibody or monoclonal antibody, or a polyspecific antibody, such as a bispecific antibody and a trispecific antibody, as long the antibody binds to a target protein. The antibody may be any antibody derived from animals, as long as the antibody exhibits a physiological effect. The antibody is preferably a human antibody, a human chimeric antibody, or a humanized antibody.

The term "antibody" according to the present disclosure typically means an immunoglobulin molecule, such as IgG, IgE, IgM, IgA, and IgD. In the present disclosure, the "antibody" includes an antibody fragment including an antigen binding region (e.g., F(ab')2 fragment, Fab' fragment, Fab fragment, Fv fragment, rIgG fragment, and a single chain antibody), and a modified antibody (e.g., a labeled antibody), as long as the antibody is able to bind to an antigen.

Examples of other forms of protein include enzyme.

Examples of the enzyme include hydrolase, phosphorylase, dephosphorylase, transferase, oxidoreductase, lyase, isomerase, and synthesized enzyme.

Examples of proteins include quercetin, testosterone, indomethacin, tranilast, and tacrolimus.

Saccharides

Examples of the saccharides include monosaccharide, disaccharide, oligosaccharide, and polysaccharide. Moreover, the saccharides also include complex carbohydrates, in which the saccharides are bonded to proteins or lipids via a covalent bond, and glycosides, in which aglycone, such as alcohol, phenol, saponin, and a dye, is bonded to a reducing group of saccharide.

Lipids

Examples of the liquids include simple lipids, complex lipids, and derived lipids.

Low Molecular Weight Compound

The low molecular weight compound typically includes a natural synthetic substance having a molecular weight of from several hundreds through several thousands. The molecular weight may be a weight average molecular weight or a number average molecular weight.

The low molecular weight compound, moreover, includes a substance equivalent to poorly water-soluble substances, and a substance equivalent to water-soluble substances.

The low molecular weight compound may be in the form of a salt, or hydrate, as long as the low molecular weight compound functions as a physiologically active substance.

The poorly water-soluble substances are not particularly limited and may be appropriately selected according to the purpose, but examples of the poorly water-soluble substances include, but are not limited to, griseofulvin, ibuprofen, itraconazole, norfloxacin, tamoxifen, cyclosporine, glibenclamide, troglitazone, nifedipine, phenacetin, phenytoin, digitoxin, nilvadipine, diazepam, chloramphenicol, indometacin, nimodipine, dihydroergotoxine, cortisone, dexamethasone, naproxen, tulobuterol, beclometasone dipropionate, fluticasone propionate, pranlukast, tranilast, loratadine, tacrolimus, amprenavir, bexarotene, calcitriol, clofazimine, digoxin, doxercalciferol, dronabinol, etoposide, isotretinoin, lopinavir, ritonavir, progesterone, saquinavir, sirolimus, tretinoin, amphotericin, fenoldopam, melphalan, paricalcitol, propofol, voriconazole, ziprasidone, docetaxel, haloperidol, lorazepam, teniposide, testosterone, valrubicin. Each of these can be used alone or in combination with others.

Examples of poorly water-soluble substances include kinase inhibitors such as gefitinib, erlotinib, osimertinib, bosnitib, vandetanib, alectinib, lorlatinib, abemaciclib, tyrophostin AG494, sorafenib, dasatinib, lapatinib, imatinib, motesanib, restaurutinib, tansutinib dorsomorphin, axitinib, 4-benzyl-2-methyl-1,2,4-thiadiazolidine-3,5-dione. Each of these can be used alone or in combination with others.

Water-soluble substances are not particularly limited and may be selected according to the purpose. Examples thereof include abacavir, acetaminophen, acyclovir, amiloride, amitriptyline, antipyrine, atropine, buspirone, caffeine, captopril, chloroquine, chlorpheniramine, cyclophosphamide, diclofenac, desipramine, diazepam, diltiazem, diphenhydramine, disopyramide, doxin, doxycycline, enalapril, ephedrine, ethambutol, ethinyl estradiol, fluoxetine, imipramine, glucose, ketorol, ketoprofen, labetalol, levodopa, levofloxacin, metoprolol, metronidazole, midazolam, minocycline, misoprostol, metformin, nifedipine, phenobarbital, prednisolone, promazine, propranolol, quinidine, rosiglitazone, salicylic acid, theophylline, valproic acid, verapamil, and zidovudine. Each of these can be used alone or in combination with others.

Physiologically Active Substances Contained in Functional Foods

The physiologically active substances contained in functional foods are not particularly limited and may be appropriately selected according to the purpose. Examples thereof include vitamin A, vitamin D, vitamin E, lutein, zeaxanthin, lipoic acid, flavonoids, and fatty acids. Each of these can be used alone or in combination with others.

Fatty acids include, for example, omega-3 fatty acids and omega-6 fatty acids.

Physiologically Active Substances Contained in Functional Cosmetics

The physiologically active substances contained in functional cosmetics are not particularly limited and may be appropriately selected according to the purpose. Examples thereof include alcohols, fatty alcohols, polyols, aldehydes, alkanolamines, alkoxylated alcohols (e.g., polyethylene glycol derivatives of alcohols and fatty alcohols), alkoxylated amides, alkoxylated amines, alkoxylated carboxylic acids, amides including salts (e.g., ceramides), amines, amino acids including salts and alkyl-substituted derivatives, esters, alkyl-substituted and acyl derivatives, polyacrylic acids, acrylamide copolymers, adipic acids copolymers, aminosilicones, biological polymers and derivatives thereof, butylene copolymers, carbohydrates (e.g., polysaccharides, chitosan, and derivatives thereof), carboxylic acids, carbomers, esters, ethers, polymer ethers (e.g., PEG derivatives, PPG derivatives), glyceryl esters and derivatives thereof, halogen compounds, heterocyclic compounds including salts, hydrophilic colloids and derivatives including salts and rubbers (e.g., cellulose derivatives, gelatin, xanthane gum, natural rubbers), imidazolines, inorganic substances (e.g., clay, $TiO_2$, ZnO), ketones (e.g., camphor), isethionates, lanoline and derivatives thereof, organic salts, phenols including salts (e.g., paraben), phosphor compounds (e.g., phosphoric acid derivatives), polyacrylates and acrylate copolymers, proteins and enzyme derivatives (e.g., collagen), synthetic polymers including salts, siloxanes and silanes, sorbitan derivatives, sterols, sulfonic acids and derivatives thereof, and waxes. Each of these can be used alone or in combination with others.

The physiologically active substance preferably has such a property that its physiological activity changes when heated, cooled, or subjected to external stress, as described above. When such physiologically active substance is included in the particles according to the present disclosure, the decrease in the amount of physiological activity is prevented in the produced particles. Therefore, based on the viewpoint that the decrease in the amount of physiological activity can be better prevented, the effect according to the present disclosure will be more pronounced when the physiologically active substance contained in the particles according to the present disclosure is a physiologically active substance whose physiological activity is easily changed by heating, cooling, or external stress. Specifically, the physiologically active substance is preferably a physiologically active substance contained in a pharmaceutical composition, more preferably at least one selected from proteins and nucleic acids, and further preferably at least one selected from antibodies and enzymes.

The content of the physiologically active substance is preferably between 1.0% by mass and 50% by mass, both inclusive, relative to the total amount of particles, and more preferably between 5.0% by mass and 40% by mass, both inclusive, relative to the total amount of particles. When the content of the physiologically active substance is between 1.0% by mass and 50% by mass, both inclusive, relative to the total amount of particles, the physiologically active substance can be encapsulated in the base material and the physiologically active substance can be gradually released.

The particles according to the present disclosure can be used, for example, in pharmaceutical compositions, functional foods, and functional cosmetics, in combination with other components such as dispersants and additives, as needed. The particles may also be functional particles for various applications. The functional particle is not particularly limited and may be appropriately selected according to the purpose. Examples thereof include prompt release particles, sustained releasing particles, pH-dependent-release particles, pH-independent-release particles, enteric coating particles, controlled-release coating particles, and nanocrystal-containing particles.

Pharmaceutical Compositions

Pharmaceutical compositions contain the particles according to the present disclosure and, if necessary, additive substances such as for formulation. The additive substances are not particularly limited and may be appropriately selected according to the purpose. Examples of such additive substances include, but are not limited to, excipients, flavoring agents, disintegrants, fluidizers, absorbents, lubricants, odor improving agents, surfactants, perfumes, coloring agents, antioxidants, masking agents, antistatic agents, and wetting agents. Each of these can be used alone or in combination with others.

Excipient

The excipient is not particularly limited and may be appropriately selected according to the purpose. Examples thereof include lactose, sucrose, mannitol, glucose, fructose, maltose, erythritol, maltitol, xylitol, palatinose, trehalose, sorbitol, crystalline cellulose, talc, silicic acid anhydride, anhydrous calcium phosphate, precipitated calcium carbonate, and calcium silicate. Each of these can be used alone or in combination with others.

Flavoring Agent

The flavoring agent is not particularly limited and may be appropriately selected according to the purpose. Examples thereof include L-menthol, refined sugar, D-sorbitol, xylitol, citric acid, ascorbic acid, tartaric acid, malic acid, aspartame, acesulfame potassium, thaumatin, saccharin sodium, dipotassium glycyrrhizinate, sodium glutamate, sodium 5'-inosinate, and sodium 5'-guanylate. Each of these can be used alone or in combination with others.

Disintegrants

The disintegrant is not particularly limited and may be appropriately selected according to the purpose. Examples thereof include low substituted hydroxypropylcellulose, carmellose, carmellose calcium, sodium carboxymethyl starch, croscarmellose sodium, crospovidone, hydroxypropyl starch, and corn starch. Each of these can be used alone or in combination with others.

Fluidizer

The fluidizer is not particularly limited and may be appropriately selected according to the purpose. Examples thereof include light anhydrous silicic acid, hydrated silicon dioxide, and talc. Each of these can be used alone or in combination with others.

As the light anhydrous silicic acid, a commercial product can be used. The commercial product thereof is not particularly limited and may be appropriately selected according to the purpose. Examples thereof include ADSOLIDER 101 (available from Freund Corporation, average pore diameter: 21 nm).

Adsorbent

As the adsorbent, a commercial product can be used. The commercial product of the adsorbent is not particularly limited and may be appropriately selected according to the purpose. Examples thereof include product name: CARPLEX (component name: synthetic silica, registered trademark of DSL. Japan Co., Ltd.), product name: AEROSIL (registered trademark of NIPPON AEROSIL CO., LTD.) 200 (component name: hydrophilic fumed silica), product name: SYLYSIA (component name: amorphous silicon dioxide, registered trademark of Fuji Silysia Chemical Ltd.), and product name: ALCAMAC (component name: synthetic hydrotalcite, registered trademark of Kyowa Chemical Industry Co., Ltd.). Each of these can be used alone or in combination with others.

Lubricant

The lubricant is not particularly limited and may be appropriately selected according to the purpose. Examples thereof include magnesium stearate, calcium stearate, sucrose fatty acid ester, sodium stearyl fumarate, stearic acid, polyethylene glycol, and talc. Each of these can be used alone or in combination with others.

Odor Improving Agent

The odor improving agent is not particularly limited and may be appropriately selected according to the purpose. Examples thereof include trehalose, malic acid, maltose, potassium gluconate, aniseed essential oil, vanilla essential oil, and cardamom oil. Each of these can be used alone or in combination with others.

Surfactant

The surfactant is not particularly limited and may be appropriately selected according to the purpose. Examples thereof include polysorbate (e.g., polysorbate 80), a polyoxyethylene-polyoxypropylene copolymer, and sodium lauryl sulfate. Each of these can be used alone or in combination with others.

Perfume

The perfume is not particularly limited and may be appropriately selected according to the purpose. Examples thereof include lemon oil, orange oil, and peppermint oil. Each of these can be used alone or in combination with others.

Coloring Agent

The coloring agent is not particularly limited and may be appropriately selected according to the purpose. Examples thereof include titanium oxide, Food Yellow No. 5, Food Blue No. 2, iron sesquioxide, and yellow iron sesquioxide. Each of these can be used alone or in combination with others.

Antioxidant

The antioxidant is not particularly limited and may be appropriately selected according to the purpose. Examples thereof include sodium ascorbate, L-cysteine, sodium sulfite, and vitamin E. Each of these can be used alone or in combination with others.

Masking Agent

The masking agent is not particularly limited and may be appropriately selected according to the purpose. Examples thereof include titanium oxide. Each of these can be used alone or in combination with others.

Antistatic Agent

The antistatic agent is not particularly limited and may be appropriately selected according to the purpose. Examples thereof include talc and titanium oxide. Each of these can be used alone or in combination with others.

Wetting Agent

The wetting agent is not particularly limited and may be appropriately selected according to the purpose. Examples thereof include polysorbate 80, sodium lauryl sulfate, sucrose fatty acid ester, macrogol, and hydroxypropyl cellulose (HPC). Each of these can be used alone or in combination with others.

The pharmaceutical composition formulations are not particularly limited and may be appropriately selected according to the purpose. Examples thereof include, but are not limited to, colon-targeted drug delivery formulations, lipid microsphere formulations, dry emulsion formulations, self-emulsification formulations, dry syrups, powder formulations for nasal or pulmonary administration, wax matrix formulations, hydrogel formulations, polymer micelle formulations, mucoadhesion formulations, gastric floating formulations, liposome formulations, and solid dispersion formulations. Each of these can be used alone or in combination with others.

The pharmaceutical composition may be in a solid dosage form such as tablet, capsule, and suppository; an aerosol for pulmonary or intranasal administration; or a liquid dosage form for injection or intraocular, intra-aural, or oral administration.

The administration route of the pharmaceutical composition is not particularly limited and may be appropriately selected according to the purpose. Examples thereof include oral administration, nasal administration, rectal administration, vaginal administration, subcutaneous administration, intravenous administration, and pulmonary administration.

Method of Producing Sustained Releasing Particle and Apparatus for Producing Sustained Releasing Particle

A method of producing a sustained releasing particle according to the present disclosure includes: discharging a liquid droplet containing a physiologically active substance, a base material, and a solvent; removing the solvent from the liquid droplet; and any other optional processes.

An apparatus of producing a sustained releasing particle includes: a liquid droplet discharger to discharge a liquid droplet containing a physiologically active substance, a base material, and a solvent, device for removing the solvent from the liquid droplet; and any other optional units.

In the present disclosure, the solvent is removed from the liquid droplet on condition that the difference ($T_d$ - $T_m$) between the dew point temperature ($T_d$) of the dry gas stream and the wet bulb temperature ($T_m$) of the discharged liquid droplets is 20 degrees Celsius or less.

Dew Point Temperature of Dry gas stream

The dew point temperature represents a temperature at which dew drop, or condensation, occurs as the gas is cooled and the air is in equilibrium with water and saturated.

The dew point temperature ($T_d$) of the dry gas stream can be obtained either by directly measuring the value with a dew point thermometer or by measuring the temperature of the dry gas stream and the relative humidity of the dry gas stream, determining the water vapor pressure (partial water vapor pressure in moist air) from those values, and determining the temperature at which the water vapor pressure is the saturated water vapor pressure.

The following calculation method is one example of calculating the dew point temperature from the temperature of the dry gas stream and the relative humidity of the dry gas stream.

The dew point temperature ($T_d$) is calculated using the following formula.

[Expression 1]

$$T_d = \frac{237.3 \log_{10}(6.1078/e)}{\log_{10}(e/6.1078) - 7.5}$$

In the above formula, "$T_d$" represents a dew point temperature [°C] and "e" represents a water vapor pressure in the air [hPa].

Water vapor pressure in the air (e), saturated water vapor pressure ($e_s$), water vapor content (absolute humidity) in the air (a), and saturated water vapor content ($a_s$) are calculated using the following equations.

[Expression 2]

$$e = e_s \times \frac{RH}{100}, \quad e_s = 6.1078 \times 10^{7.5t/(t+237.3)}$$

[Expression 3]

$$a = a_s \times \frac{RH}{100}, \quad a_s = \frac{217 \times e_s}{t + 273.15}$$

In the above formula, "e" represents a water vapor pressure in the air [hPa], "$e_s$" represents a saturated water vapor pressure [hPa], "a" represents a water vapor content of air (absolute humidity) [g/m$^3$], "$a_s$" represents a saturated water vapor content [g/m$^3$], "RH" represents a relative humidity [%], and "t" represents a temperature [°C].

Wet Bulb Temperature of Discharged (Ejected) Liquid Droplets

The wet bulb temperature is the temperature a given air mass would have if the air mass were cooled adiabatically until reaching a saturated state by vaporizing water in the air mass while maintaining a constant atmospheric pressure.

The wet bulb temperature ($T_m$) of the ejected liquid droplet is calculated by the following formula.

[Expression 4]

$$\Delta h\{Y_m - Y\} = C_H(T - T_m)$$

In the above formula, "$\Delta h$" represents a latent heat of vaporization of the solvent in the liquid droplet [kJ/kg], "$Y_m$" represents a humidity [weight of vapor (kg)/weight of dry gas (kg)] when the solvent in the liquid droplet is saturated at the wet bulb temperature, "Y" represents a humidity [weight of vapor (kg)/weight of dry gas (kg)], "$C_H$" represents a specific heat capacity of wet gas [kJ/kg-K], "T" represents a gas streams temperature [°C], and "$T_m$" represents a wet bulb temperature of the liquid droplet [°C].

Here, the specific heat capacity ($C_H$) of the wet gas can be calculated by the following formula.

$$[Expression\ 5]$$

$$C_H = C_G + C_v \cdot Y$$

In the above formula, "$C_H$" represents a specific heat capacity of the wet gas [kJ/kg·K], "$C_G$" represents a specific heat capacity of the gas [kJ/kg·K], "Cv" represents a specific heat capacity of the solvent vapor [kJ/kg-K], and "Y" represents a humidity [weight of vapor (kg)/weight of dry gas (kg)].

The humidity Y can be calculated by the following formula, and $Y_m$ can be calculated by substituting the vapor pressure [Pa] of the solvent in the liquid droplet at the wet bulb temperature $T_m$ for "$p_v$" in the following formula.

$$[Expression\ 6]$$

$$Y = \frac{M_V \cdot p_V}{M_G (p - p_v)}$$

In the above formula, "Y" represents a humidity [weight of vapor (kg)/weight of dry gas (kg)], "$M_v$" represents a molar mass of vapor [kg/mol], "$M_G$" represents a molar mass of gas [kg/mol], "p" represents a pressure of dry gas stream [Pa], and "$p_v$" represents a vapor pressure of solvent in the liquid droplet [Pa].

The vapor pressure of the solvent in the liquid droplet can be the value shown in the Chemistry Handbook, Basic Edition (edited by the Chemical Society of Japan, Maruzen Publishing Co., Ltd.) or in textbooks, or can be calculated using a predictive equation such as the Antoine equation.

The difference ($T_d$ - $T_m$) between the dew point temperature ($T_d$) of the dry gas stream and the wet bulb temperature ($T_m$) of the ejected liquid droplet is not particularly limited and may be appropriately selected according to the purpose, as long as it is 20°C or below, but 10°C or below is preferred.

As the difference $T_d$ - $T_m$ increases, moisture in the dry gas stream condenses on the particle surface, slowing the drying rate of the particles. The slower drying of the particles allows more time for the physiologically active substance in the liquid droplet to migrate to the surface, which increases the amount of physiologically active substance on the particle surface and contributes to the initial burst increase.

Particularly when the physiologically active substance is a water-soluble compound, the distribution of the physiologically active substance toward the surface is likely to be more pronounced due to the increased water content on the particle surface.

The temperature of the dry gas stream is not particularly limited and may be appropriately selected according to the purpose, as long as $T_d$ - $T_m$ is 20°C or below, and maybe for example, 26°C to 60°C.

The relative humidity of the dry gas stream is not particularly limited and may be appropriately selected according to the purpose, as long as $T_d$ - $T_m$ is 20°C or below, and may be for example, 2.1% to 35%.

In the present disclosure, "removal" means that the solvent contained in the liquid phase is removed from the liquid phase. However, the present disclosure is not limited to the case where all the solvent contained in the liquid phase is removed, and may also include such cases where the solvent contained in the liquid phase remains, as long as particles can be prepared. Moreover, "removal" in the present disclosure is not particularly limited as long as a solvent is removed from a liquid droplet under conditions where the difference ($T_d$ - $T_m$) between the dew point temperature ($T_d$) of the dry gas stream and the wet bulb temperature ($T_m$) of the discharged liquid droplet is 20°C or below. For example, "removal" in the present disclosure may include vaporization of a solvent from the liquid phase in a gas or in vacuo (hereinafter referred to as "air drying ").

The drying rate of a liquid droplet is expressed by the following formula.

$$[Expression\ 7]$$

$$J = k_H (Y_m - Y)$$

In the above formula, "J" represents a drying rate [amount of solvent to be vaporized (kg)/vaporization area ($m^2$)/time (s)] and "$K_H$" represents a mass transfer constant [kg/$m^2$/s].

The drying rate is proportional to the value of $Y_m$-Y. When the drying rate is fast, the physiologically active substance in the liquid droplet has less time to move to the surface, thus reducing the initial burst. The difference ($Y_m$-Y) between the humidity ($Y_m$) when the solvent is saturated in the liquid droplet at the wet bulb temperature of the dry gas stream and the humidity (Y) of the dry gas stream is not particularly limited and may be appropriately selected according to the purpose, but 0.07 or more is preferred and 0.08 or more is more preferred. The drying rate varies not only with the conditions of the dry gas stream but also with the composition of the solvent in the liquid phase, and a solvent composition with a high vapor pressure can be used to increase the drying rate.

Next, an example of a case in which the removal is conducted as the air drying is described as the present embodiment, but the method of producing particles and the apparatus for producing particles are not limited to such embodiment.

Method of Producing Particles

The method of producing particles using the air drying method includes: a liquid droplet discharge step of discharging, into air, liquid droplets containing a physiologically active substance, a base material, and a solvent; a granulating step of preparing particles by vaporizing the solvent from the liquid droplets and removing the solvent contained in the liquid droplets; and any other step as necessary.

Several dry granulation methods are conventionally known for preparing particles in the air.

One type of method is an air milling method exemplified by: a method in which particle materials are melted, kneaded, and uniformly dispersed to obtain a molten mixture, then the molten mixture is cooled and milled using a milling machine to obtain small-sized milled particles; and a method in which a liquid containing particle materials is freeze-dried and then milled using a milling machine to obtain small-sized milled particles.

Another type of method is a spray drying method (spray drying method), exemplified by a method in which a liquid containing particle materials is sprayed into the air and dried to obtain small-sized sprayed particles. There are several spraying methods, including a pressurized nozzle type, in which liquid is pressurized and ejected from a nozzle, and a disk type, in which liquid is sent to a high-speed rotating disk and scattered by centrifugal force.

In the air milling method, while the equipment used therefor when milling is simple, it is difficult to produce particles with a narrow particle size distribution.

In addition, in the method of milling molten mixtures, if a physiologically active substance having such a property that its physiological activity changes when heated is included as a material of the particles, the physiological activity of that physiologically active substance changes, resulting in a decrease in the amount of physiological activity.

In the method of milling freeze-dried materials, if a physiologically active substance having such a property that its physiological activity changes when cooled is included as a material of particles, the physiological activity of that physiologically active substance changes, resulting in a decrease in the amount of physiological activity.

Furthermore, in the air milling method, large external stress is generated during the milling process. Therefore, if a physiologically active substance having such a property that its physiological activity changes when subjected to external stress is included as a material of particles, the physiological activity of that physiologically active substance changes, resulting in a decrease in the amount of physiological activity.

While the spray drying method can produce particles having a high ratio of the physiologically active substance retained in the particles (physiologically active substance retention rate) after the particle production, it is generally difficult to produce particles with a small particle size.

The disk type spraying method may be able to produce particles with a small particle size, but requires extensive equipment.

In the spray drying method, liquid droplets tend to coalesce in the air, making it difficult to produce particles with a narrow particle size distribution. In order to prevent liquid droplets from coalescing with each other in the air, it is necessary to heat the liquid droplets so that the sprayed liquid droplets dry immediately. However, if a physiologically active substance having such a property that its physiological activity changes when heated is included as a particle material, the physiological activity of that physiologically active substance changes, resulting in a decrease in the amount of physiological activity.

Furthermore, in the spray drying method, large external stress is generated by spraying with high shearing force. Therefore, if a physiologically active substance having such a property that its physiological activity changes when subjected to external stress is included as a particle material, the physiological activity of that physiologically active substance changes, resulting in a decrease in the amount of physiological activity.

The production method of the particles according to the present embodiment described below does not correspond to the above-described air milling method and spray drying method. Therefore, even when a physiologically active substance having such a property that its physiological activity changes when heated, cooled, or subjected to external stress is contained as a material of the particles, the change in the physiological activity of the physiologically active substance is prevented, and as a result, the decrease in the amount of physiological activity is also prevented.

In addition, as described below, the production method of the particles according to the present embodiment preferably does not include a step of using a unit such as shaking or stirring for applying external stress that changes the physiological activity of the physiologically active substance, a step of using a heater heating to a temperature at which the physiological activity of the physiologically active substance changes, or a step of using a cooler cooling to a temperature at which the physiological activity of the physiologically active substance changes.

Liquid Droplet Discharge Step

The liquid droplet discharge step is a step of discharging, into a gas, liquid droplets containing a physiologically active substance, a base material, and a solvent.

The method for discharging liquid droplets is not particularly limited, and examples thereof include the following methods.

(I) A method using a discharger that discharges a pressurized liquid as liquid droplets from a hole provided on a plate-like nozzle forming surface such as an inkjet nozzle.

(ii) A method using a discharger that discharges a pressurized liquid as liquid droplets from a hole formed in an irregular shape such as an SPG membrane

(iii) A method using a discharger that discharges a liquid to which vibration is applied from a hole as liquid droplets

Examples of the discharger which utilizes vibration in (iii) stated above and which does not change the physiological activity of the physiologically active substance by the vibration include a unit which hardly applies external stress to the particle composition liquid itself, such as a discharger utilizing a membrane vibration method, a discharger utilizing a Rayleigh fission method, a discharger utilizing a liquid vibration method, and a discharger utilizing a liquid column resonance method. In addition, these dischargers may further include a unit applying pressure to the liquid to discharge the liquid. Among these units, a discharger using a liquid column resonance method which further has a unit that discharges a liquid by applying a pressure to the liquid is preferable.

Examples of the discharger using the liquid column resonance method include a discharger using a method in which a standing wave is formed by liquid column resonance by applying vibration to a liquid accommodated in a liquid-column-resonant liquid chamber and the liquid is discharged from discharge holes formed in an area corresponding to an antinode of the standing wave in an amplitude direction of the standing wave.

An example of the discharger using the membrane vibration method is a discharger including a thin film on which a plurality of discharge holes are formed, and an annular vibration generator which is disposed around a deformable region of the thin film and vibrates the thin film.

Examples of the discharger using the Rayleigh fission method include a discharger which supplies a liquid to a storage portion, discharges the liquid from a plurality of through holes provided in the storage portion while applying vibration to the storage portion by a vibrator in contact with a part of the storage portion, and forms the raw material fluid into liquid droplets from a columnar state through a constricted state.

Examples of the discharger using the liquid vibration method include a discharger having a step of supplying a liquid to a storage member having a film in which a plurality of discharge holes are formed, and a step of vibrating the liquid supplied to the storage member to form liquid droplets from the plurality of discharge ports.

As an example of the liquid droplet discharge step, a method of discharging, as liquid droplets, a liquid containing a base material, a physiologically active substance, and a solvent, by vibration, is described as follows.

The discharging method by vibration is not particularly limited, and examples thereof include the following methods. Each method is described below.

(a) Method using a volume changer for changing the volume of a liquid containing portion using vibration

(b) Method using a constriction generator discharging the liquid from a plurality of discharge holes provided in the liquid containing portion while applying vibration to the liquid containing portion to form the liquid into liquid droplets from a columnar state through a constricted state.

(c) Method using a nozzle vibrator vibrating a thin film on which a nozzle is formed

The volume changer is not particularly limited and may be appropriately selected according to the purpose as long as the volume of the liquid containing portion can be changed. Examples of the volume changer include a piezoelectric element (also referred to as a "piezo element") that expands and contracts when a voltage is applied thereto.

Examples of the constriction generator include a unit which discharges the liquid from a plurality of nozzle holes provided in the liquid containing portion while applying vibration to the liquid containing portion by a vibrator using a piezoelectric element which is in contact with a part of the liquid containing portion, and forms the liquid into liquid droplets from a columnar state through a constricted state.

Examples of the nozzle vibrator include a unit which discharges a liquid from a plurality of nozzle holes provided in the liquid containing portion by using a thin film on which the plurality of nozzles are formed and a piezoelectric element which is disposed around a deformable region of the thin film and vibrates the thin film, thereby to form liquid droplets.

As a unit that generates vibration, a piezoelectric element is generally used. The piezoelectric element is not particularly limited, and the shape, size, and material thereof may be appropriately selected according to the purpose. For example, a piezoelectric element used in a conventional inkjet discharge method can be suitably used.

The shape and size of the piezoelectric element are not particularly limited, and may be appropriately selected in accordance with the shape of the discharge hole and the like.

The material of the piezoelectric device is not particularly limited and may be appropriately selected according to the purpose. Examples thereof include piezoelectric ceramics such as lead zirconate titanate (PZT), piezoelectric polymers such as polyvinylidene fluoride (PVDF), crystals, and single crystals of $LiNbO_3$, $LiTaO_3$, and $KNbO_3$.

The discharge hole is not particularly limited and may be appropriately selected according to the purpose. Examples thereof include an opening provided in a nozzle plate or the like.

The cross-sectional shape and size of the discharge hole may be appropriately selected according to the purpose.

The cross-sectional shape of the discharge hole is not particularly limited and may be appropriately selected according to the purpose. Examples thereof include: (1) a tapered shape in which the opening diameter decreases from the inner side (liquid containing portion side) toward the outside (liquid discharge side); (2) a shape in which the opening diameter decreases while having a round shape from the inner side (liquid containing portion side) toward the outside (liquid discharge side); (3) a shape in which the opening diameter decreases at a certain nozzle angle from the inner side (liquid containing portion side) toward the outside (liquid discharge side); and (4) a combination of the shape of (1) and the shape of (2). Among these, the shape of (3) is preferable from the viewpoint that the pressure applied to the liquid in the discharge hole is maximized.

The nozzle angle in the shape of (3) is not particularly limited and may be appropriately selected according to the purpose, but is preferably 60 degrees or more and 90 degrees or less. When the nozzle angle is 60 degrees or more and 90 degrees or less, the liquid droplet can be stably discharged.

The size of the discharge hole is not particularly limited and may be appropriately selected according to the purpose. For example, the diameter of the discharge hole is preferably less than 1000 μm, more preferably 1.0 μm or more and less than 1000 μm, further preferably 1.0 μm or more and 500 μm or less, particularly preferably 1.0 μm or more and 50 μm or less. When the shape of the discharge hole is not a true circle, the diameter of a true circle having an area equivalent to the area of the discharge hole is adopted.

Particle Composition Liquid

The liquid (particle composition liquid) discharged in the liquid droplet discharge step contains a physiologically active substance, a base material, and a solvent. The base material and the physiologically active substance contained in the particle composition liquid may be various materials similar to the base material and the physiologically active substance contained in the particles, and thus the description thereof will be omitted, and only the solvent will be described.

The content of the physiologically active substance or the base material in the particle composition liquid is not particularly limited and may be appropriately selected according to the purpose.

It is preferable that the particle composition liquid and the solvent do not substantially contain a surfactant. In a case where the particle composition liquid and the solvent contain substantially no surfactant, a pharmaceutical composition, a functional food, a functional cosmetic, or the like, which contains the produced particles, have improved safety. Here, examples of the case where the particle composition liquid and the solvent contain substantially no surfactant includes, but are not limited to, a case where the content of the surfactant in the particle composition liquid and the solvent is equal to or less than the detection limit at which the surfactant is undetectable by liquid chromatography, and a case where the particle composition liquid and the solvent contain no surfactant.

Solvent

The solvent is a liquid in which the base material is dissolved. The solvent may be a liquid in which a physiologically active substance can also be dissolved, but is preferably a liquid in which a physiologically active substance is dispersed.

The state in which a physiologically active substance is dissolved means a state in which the physiologically active substance is dissolved in a liquid to form a uniform liquid. In addition, the state in which the physiologically active substance is dispersed means a state in which the physiologically active substance is not dissolved in a liquid but is present in the liquid in the form of a solid or an emulsion.

Examples of the solvent include, water, halogenated aliphatic hydrocarbons (e.g., dichloromethane, dichloroethane, and chloroform), alcohols (e.g., methanol, ethanol, and propanol), ketones (e.g., acetone and methyl ethyl ketone), ethers (e.g., diethyl ether, dibutyl ether, and 1, 4-dioxane), aliphatic hydrocarbons (e.g., n-hexane, cyclohexane, and n-heptane), aromatic hydrocarbons (e.g., benzene, toluene, and xylene), organic acids (e.g., acetic acid and propionic acid), esters (e.g., ethyl acetate), amides (e.g., dimethylformamide and dimethylacetamide), or mixed solvents thereof. Each of these can be used alone or in combination with others. Among these, halogenated aliphatic hydrocarbons, alcohols, or mixed solvents thereof are preferable from the viewpoint of solubility, and dichloromethane, 1,4-dioxane, methanol, ethanol, acetonitrile, or mixed solvents thereof are more preferable.

The content of the solvent is preferably from 70% by mass to 99.5% by mass, both inclusive, and more preferably from 90% by mass to 99% by mass, both inclusive, relative to the mass of the particle composition liquid. When the content is 70% by mass or more and 99.5% by mass or less, the production stability improves in terms of the solubility of the particulate material and the liquid viscosity.

The viscosity of the particle composition liquid is not particularly limited and may be appropriately selected according to the purpose, but is preferably 0.5 mPa s or more and 15.0 mPa s or less, more preferably 0.5 mPa s or more and 10.0 mPa s or less. The viscosity can be measured, for example, using a viscoelasticity measuring apparatus (apparatus name: MCR Rheometer, available from Anton Paar) under the conditions of 25 degrees Celsius and shear rate $10s^{-1}$. The viscosity of the liquid being 0.5 mPa·s or more and 15.0 mPa·s or less is preferable because then the above-stated liquid droplet discharger can perform suitable discharge.

The surface tension of the particle composition liquid is not particularly limited and may be appropriately selected according to the purpose, but is preferably 10 mN/m or more and 60 mN/m or less, more preferably 20 mN/m or more and 50 mN/m or less. The surface tensions can be measured, for example, using a portable surface tensiometer (device name: PocketDyne, available from KRUSS) by the maximum foaming pressure method under conditions of 25 degrees Celsius and lifetimes of 1,000 ms. The liquid having a surface tension of 0.5 mPa·s or more and 15.0 mPa·s or less is preferable because then the above-stated liquid droplet discharger can perform suitable discharge.

Removing

The solvent of a liquid droplet is removed by vaporizing the solvent from the liquid droplet to remove the solvent contained in the liquid droplet.

The removing is performed in a gas. Specifically, removing is preferably performed while the liquid droplets discharged into the gas in the liquid droplet discharge step are flying in the gas.

By the removing, a particle in which the physiologically active substance is dispersed in the base material can be produced.

Unlike the conventional spray drying method, the particles produced by the present method do not require drying involving heating or cooling. Therefore, the present method are particularly advantageous for forming particles each containing a physiologically active substance whose physiological activity isreasily changed by heating or cooling.

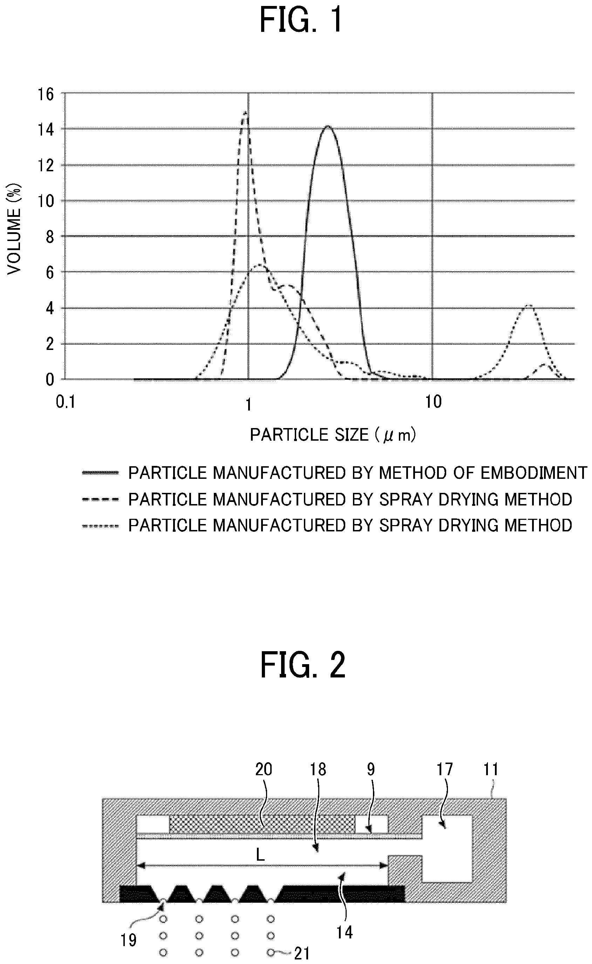

In addition, since it is possible to discharge liquid droplets having a substantially uniform size while controlling the liquid droplets so as not to coalesce each other, and to perform preparation by vaporizing the solvent from the liquid droplets, a large amount of particles having a uniform size can be produced, and accordingly the particle size distribution can be narrowed, as illustrated in FIG. 1.

FIG. 1 illustrates an exemplary particle size distribution of particles produced in the method according to the present embodiment and particles produced by a spray drying method. According to FIG. 1, unlike the particles produced by the spray drying method, the particles produced by the method of the present embodiment have only one narrow peak in the particle size distribution, and do not have any peak representing coarse particles.

In addition, the particle size of the particles can also be adjusted by appropriately adjusting the size or the like of the discharge hole of the discharger forming the liquid droplets.

Further, as a unit that reduces the particle size, a discharger forming liquid droplets by vibration or the like is used instead of a pulverizing apparatus generating large external stress or a spraying apparatus applying high shearing force. Accordingly, even when a physiologically active substance having such a property that its physiological activity changes when subjected to external stress is contained as a material of the particles, the change of the physiological activity of the physiologically active substance is prevented, and as a result, the decrease in the amount of the physiological activity can be prevented.

In addition, since contact with a solvent such as water is not required during preparation according to the present step, particles having a high ratio of the physiologically active substance retained in the particles (physiologically active substance retention rate) after the particle production step can be produced. The physiological activity ratio of the particles produced according to the present step improves as compared with those produced according to other methods. For example, the physiological activity ratio of the particles produced according to the present step is 50% or more.

In the removing , the solvent may be vaporized from the liquid droplets by discharging the liquid droplets into the conveyance gas stream to prepare the particles. A method of vaporizing the solvent from the liquid droplets by using the conveyance gas stream is not particularly limited and may be appropriately selected according to the purpose. For example, a method of setting the conveyance direction of the conveyance gas stream to be substantially perpendicular to the direction in which the liquid droplets are discharged is preferable.

In addition, the temperature, the vapor pressure, the type of gas, and the like, of the conveyance gas stream are preferably appropriately adjusted. Note that a heater may be provided to adjust the temperature of the conveyance gas stream, but as described above, in the removing, discharge is performed in which coalescence of the liquid droplets is prevented. Therefore, the degree of heating by the heater can be lowered, and specifically, the heating can be performed to such an extent that the physiological activity of the physiologically active substance does not change.

In addition, as long as the collected particles maintain a solid state, the solvent does not have to be completely vaporized, and a drying step may be additionally provided as a separate step after the collection. In addition, a method of vaporizing the solvent from the liquid droplets by applying a temperature change or a chemical change may be used.

Other Steps

Other steps are not particularly limited and may be appropriately selected according to the purpose. Examples of other steps include a particle collecting step.

The particle collecting step is a step of collecting the produced particles, and can be suitably performed by a particle collector. The particle collector is not particularly limited and may be appropriately selected according to the purpose. Examples of the particle collector include a cyclone collector and a bag filter.

In the case of producing particles containing at least two types of base materials in which one base material of the at least two types of base materials is distributed more toward the surface side, the above-mentioned type of particles can be formed in the removing by appropriately selecting the type of the base material contained in the particle composition liquid.

In the removing step, in order to form particles in which one of the at least two types of base materials is distributed more toward the surface side, the contact angles of the at least two types of base materials are made different from each other. Thus, when the solvent is vaporized from the liquid droplets in the removing, the interaction between the base materials increases.

At this time, since the at least two types of base materials have different contact angles from each other, the base materials are easily phase-separated from each other. Then one of the at least two types of base materials is distributed more toward the surface side, and thereafter, when vaporization of the solvent proceeds, particles solidified in that state are formed. By this method, one type of particles in which one base material of at least two base materials is distributed more toward the surface side by only one step.

The difference between the contact angles of the base materials is not particularly limited and may be appropriately selected according to the purpose. The difference is preferably 1.0 degrees or more, more preferably 10.0 degrees or more. When the difference between the contact angles of the base materials is within the preferable range, the base materials are easily phase-separated from each other.

A method of measuring the contact angle of the base material is not particularly limited and may be appropriately selected according to the purpose. Examples of the method include a method of measuring the contact angle with a contact angle meter. Examples of the contact angle meter include a portable contact angle meter, PG-X+/mobile contact angle analyzer, available from FIBRO System.

A method for confirming the presence or absence of phase separation in the at least two types of base materials is not particularly limited and may be appropriately selected according to the purpose. Examples of the method include a method in which a solution obtained by dissolving the at least two types of base materials in a solvent is formed into a thin film with a bar coater, and states before drying, during drying, and after drying are confirmed with an optical microscope. Examples of the optical microscope include OLYMPUS BX51 available from Olympus Corporation.

Further, in the case where the solvent used together with the base material is lipophilic, one base material to be distributed more toward the surface side is selected to have a contact angle larger than that of the other base material to be distributed more toward the inner side of the particle. Since the one base material having a larger contact angle has a greater affinity for the solvent than the other base material distributed more toward the inner side of the particle has, the one base material tends to be distributed more toward the solvent side, that is, toward the surface side.

In addition, when the solvent used together with the base material is hydrophilic, one base material to be distributed more toward the surface side is selected so as to have a contact angle smaller than that of the other base material to be distributed more toward the inner side of the particle. Since the one base material having a small contact angle has a smaller affinity for the solvent than the other base material distributed more toward the inner side of the particle, the one base material is easily distributed more toward the solvent side, that is, toward the surface side.

Therefore, for example, when it is desired to produce particles containing a water-soluble physiologically active substance as in many pharmaceutical compositions, the solvent may be made lipophilic, to obtain a structure in which the physiologically active substance is distributed more toward the inner side of the particle and the inner side is coated with one base material distributed more toward the surface side.

When it is desired to produce particles containing an oil-soluble physiologically active substance, the solvent may be made hydrophilic, to obtain a structure in which the physiologically active substance is distributed more toward the inner side of the particle and the inner side is coated with one base material distributed more toward the surface side.

Examples of the base material to be distributed more toward the surface side include a pH-responsive material. By using such pH-responsive material, for example, particles that can be contained in an enteric pharmaceutical composition can be produced.

Examples of the pH -responsive material include cellulose-based polymers and methacrylic acid-based polymers. Since the cellulose-based polymers and the methacrylic acid-based polymers have a larger contact angle than other base materials, it is possible to form a particle having a structure in which the physiologically active substance is distributed more toward the inner side of the particle and the inner side is coated with one base material distributed more toward the surface side. These may be used alone, or two or more types thereof may be used together.

Among the cellulose-based polymers, hydroxypropylmethyl cellulose acetate succinate and hydroxypropylmethyl cellulose phthalate are preferable. Since these materials have a larger contact angle than other base materials, a particle can be formed which has a structure in which a physiologically active substance is distributed more toward the inner side of the particle and the inner side is coated with one base material distributed more toward on the surface side. These may be used alone, or two or more types thereof may be used together.

Among the methacrylic acid-based polymers, an ammonioalkyl methacrylate copolymer is preferable. This is because the contact angle of an ammonioalkyl methacrylate copolymer is larger than those of other base materials.

As a combination of one base material to be distributed more toward the surface side and another base material to be distributed more toward the inner side, for example, a combination of any one selected from poly (meth) acrylic acid, polyglycolic acid, and hydroxypropyl methylcellulose and any one selected from hydroxypropyl cellulose, polyethylene pyrrolidone, and polyalkylene glycol is preferable. This is because these combinations are not compatible with each other and are phase-separated.

Examples of the other steps include, in addition to those described above, a step of obtaining particles having a uniform size by filtering or sieving particles after removing.

Particle Production Apparatus

The particle production apparatus includes: a liquid droplet discharger that discharges liquid droplets containing a physiologically active substance, a base material and a solvent into a gas; a granulation device that prepares particles by vaporizing the solvent from the liquid droplets and removing the solvent contained in the liquid droplets; and any other units as necessary.

Liquid Droplet Discharger

The liquid droplet discharger is a unit that discharges a liquid containing a physiologically active substance, a base material, and a solvent into a gas to form liquid droplets.

In a preferred embodiment, the liquid droplet discharger discharges the particle composition liquid by vibration to form liquid droplets.

The liquid droplet discharger is connected to a liquid container described below. The unit that connects the liquid droplet discharger and the liquid container is not particularly limited and may be appropriately selected according to the purpose, as long as the liquid can be supplied from the liquid container to the liquid droplet discharger. Examples thereof include a tube (e.g., a pipe and a tube).

The liquid droplet discharger preferably has a vibration applying member that applies vibration to the liquid to discharge the liquid droplets. The vibration is not particularly limited and may be appropriately selected according to the purpose. For example, the frequency of the vibration is preferably 1 kHz or more, more preferably 150 kHz or more, and further preferably from 300 to 500 kHz, both inclusive. When the frequency is 1 kHz or more, a liquid column ejected from the discharge hole can be made into liquid droplets with good reproducibility. When the frequency is 150 kHz or more, production efficiency can be improved.

Examples of the liquid droplet discharger having the vibration applying member include an ink jet nozzle. Examples of a discharge mechanism of the inkjet nozzle include, but not limited to, a liquid column resonance method, a membrane vibration method, a liquid vibration method, and a Rayleigh fission method.

Next, a liquid-column-resonant liquid droplet discharger will be specifically described as an example of the liquid droplet discharger.

It should be understood by those skilled in the art that the liquid droplet discharger is not limited to the liquid-column-resonant liquid droplet discharger, and other liquid droplet dischargers (for example, a discharger using a membrane vibration method, discharger using a Rayleigh fission method, and discharger using a liquid vibration method) may be used.

FIG. 2 is a schematic cross-sectional view of an example of a liquid-column-resonant liquid droplet discharger. The liquid-column-resonant liquid droplet discharger 11 has a liquid common supply channel 17 and a liquid-column-resonant liquid chamber 18. The liquid-column-resonant liquid chamber 18 communicates with the liquid common supply channel 17 disposed on one of both longitudinal end wall surfaces. The liquid-column-resonant liquid chamber 18 has discharge holes 19 that discharge liquid droplets 21, on one wall surface thereof which is connected with its both longitudinal end wall surfaces. The liquid-column-resonant liquid chamber 18 also has a vibration generator 20 that generates high-frequency vibration for forming a liquid-column-resonant standing wave, on the wall surface facing the discharge holes 19. The vibration generator 20 is connected to a high-frequency power source. Further, a gas stream passage may be provided that supplies gas streams for conveying the liquid droplets 21 discharged from the liquid-column-resonant liquid droplet discharger 11.

A liquid 14 containing a physiologically active substance, a base material, and a solvent is let to flow into the liquid common supply channel 17 in the liquid-column-resonant liquid droplet discharger 11 through a liquid supply tube by a liquid circulating pump and is supplied to the liquid-column-resonant liquid chamber 18.

Within the liquid-column-resonant liquid chamber 18 filled with the liquid 14, the vibration generator 20 generates a liquid-column-resonant standing wave, thereby forming a pressure distribution. The liquid droplets 21 are discharged from the discharge holes 19 provided within an area corresponding to an antinode of the liquid-column-resonant standing wave where the amplitude is large and pressure variation is large.

The area corresponding to an antinode of the liquid-column-resonant standing wave is an area not corresponding to a node of the standing wave. Preferably, the area corresponding to the antinode is an area where the amplitude in pressure variation of the standing wave is large enough to discharge liquid, more preferably, an area extending from a position at a local maximum amplitude of a pressure standing wave (i.e., a node of a velocity standing wave) toward a position at a local minimum amplitude for a distance of one-quarter wavelength.

Within the area corresponding to an antinode of the standing wave, even in a case in which multiple discharge holes are provided, each of the multiple discharge holes can discharge substantially uniform liquid droplets efficiently without causing clogging. Note that the liquid 14 having passed through the liquid common supply channel 17 is circulated by a liquid return tube 322. As the liquid droplets 21 are discharged, the amount of the liquid 14 in the liquid-column-resonant liquid chamber 18 is reduced and a suction force generated by the action of the liquid-column-resonant standing wave is also reduced within the liquid-column-resonant liquid chamber 18. Thus, the flow rate of the liquid 14 supplied from the liquid common supply channel 17 increases. Then the liquid-column-resonant liquid chamber 18 is refilled with the liquid 14. After the liquid-column-resonant liquid chamber 18 is refilled with the liquid 14, the flow rate of the liquid 14 passing within the liquid common supply channel 17 is returned to a former state.

The liquid-column-resonant liquid chamber 18 in the liquid-column-resonant liquid droplet discharger 11 may be formed of joined frames made of a material having a high stiffness that does not adversely affect resonant frequency of the liquid at drive frequency of metals, ceramics, and silicone, for example.

A length L between the wall surfaces of the liquid-column-resonant liquid chamber 18 at both longitudinal ends thereof, illustrated in FIG. 2, is determined based on a mechanism of liquid column resonance.

Preferably, multiple liquid-column-resonant liquid chambers 18 are provided to a single liquid droplet formation unit to drastically improve productivity.

The number of the liquid-column-resonant liquid chambers 18 is not particularly limited, but is preferably from 1 to 2,000, both inclusive.

Each of the liquid-column-resonant liquid chambers 18 is communicated with the liquid common supply channel 17 through each liquid supply channel. The liquid common supply channel 17 is communicated with multiple liquid-column-resonant liquid chambers 18.

The vibration generator 20 in the liquid-column-resonant liquid droplet discharger 11 is not limited to any particular device as long as it can be driven at a specific frequency. For example, the vibration generator 20 may be formed of a piezoelectric body and an elastic plate 9 attached to each other.

The drive frequency of the vibration generator 20 is preferably 150 kHz or more, more preferably from 300 to 500 kHz, both inclusive, for improving productivity.

The elastic plate 9 constitutes a part of the wall of the liquid-column-resonant liquid chamber 18 so that the piezoelectric body does not contact the liquid.

The piezoelectric body may be made of a piezoelectric ceramic, such as lead zirconate titanate (PZT), which is generally laminated because of having a small displacement. The piezoelectric body may also be made of a piezoelectric polymer such as polyvinylidene fluoride (PVDF), crystal, or a single crystal of $LiNbO_3$, $LiTaO_3$, or $KNbO_3$.

Preferably, the vibration generator 20 is disposed in each liquid-column-resonant liquid chamber 18 so as to control each liquid-column-resonant liquid chamber 18 independently.

Preferably, a single blockish vibrating material may be partially cut to fit the arrangement of the liquid-column-resonant liquid chambers 18 so as to control each liquid-column-resonant liquid chamber 18 independently through the elastic plate 9.

Preferably, the discharge holes 19 are arranged in a width direction in the liquid-column-resonant liquid chamber 18, so that a large number of discharge holes 19 can be provided for improving production efficiency.

Since the liquid column resonant frequency varies depending on the arrangement of the opening of the discharge holes 19, the liquid column resonant frequency is preferably adjusted monitoring the discharge condition of liquid droplets.

Liquid Container

The liquid container is a container storing a liquid containing a physiologically active substance, a base material, and a solvent.

The liquid container may or may not have flexibility. The material of the liquid container is not particularly limited and may be appropriately selected according to the purpose. For example, the liquid container may be made of a resin or a metal. The structure of the liquid container is not particularly limited and may be appropriately selected according to the purpose. For example, the liquid container may be a sealed container or a non-sealed container.

Granulation Device

The granulation device prepares particles by vaporizing the solvent from the liquid droplets to remove the solvent contained in the liquid droplets.

Examples of the granulation device include a member that forms a space for vaporizing the solvent from the liquid droplets.

The granulation device preferably has a conveyance gas stream former that forms the conveyance gas stream.

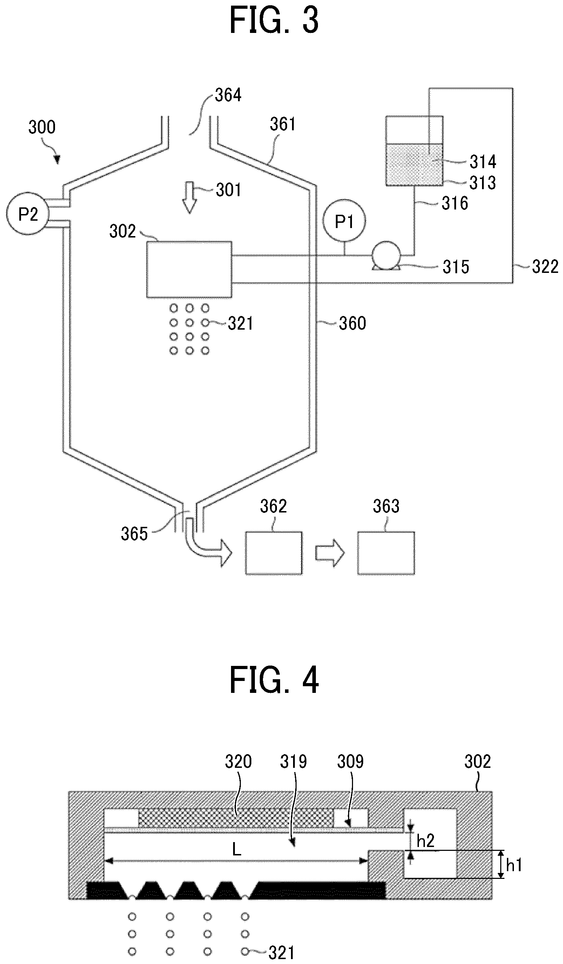

Next, an example of the embodiment will be described with reference to FIGs. 3 to 5.

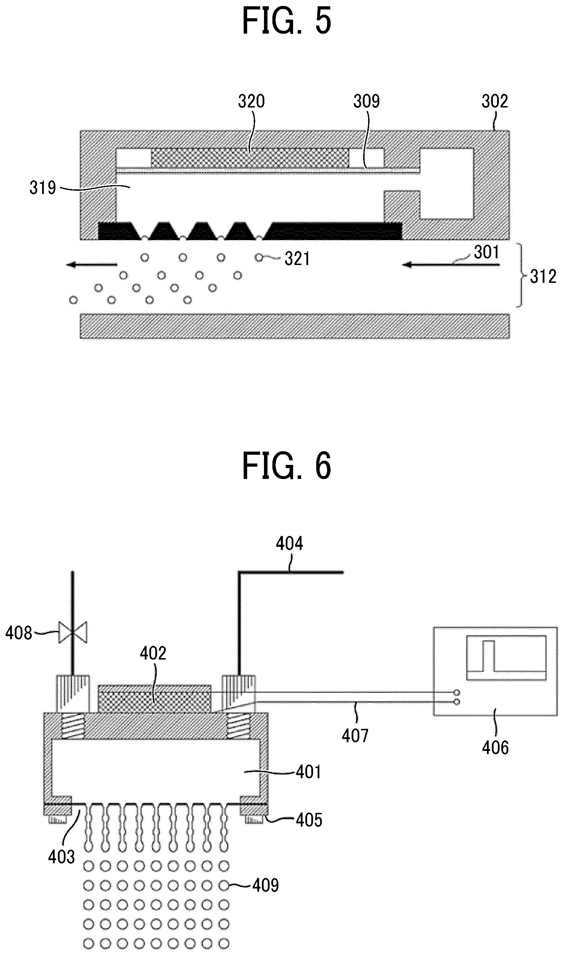

FIG. 3 is a schematic diagram illustrating an example of a particle production apparatus. FIG. 4 is a schematic cross-sectional view of an example of a liquid droplet discharger used in the particle production apparatus. FIG. 5 is a schematic cross-sectional view of another example of the liquid droplet discharger used in the particle production apparatus.

The particle production apparatus 300 illustrated in FIG. 3 includes a liquid droplet discharger 302, a drying collecting unit 360, a conveyance gas stream discharge port 365, and a particle storage portion 363. The liquid droplet discharger 302 is connected to a liquid container 313 that contains the liquid 314, and a liquid circulating pump 315 that supplies the liquid 314 contained in the liquid container 313 to the liquid droplet discharger 302 through a liquid supply tube 316 and returns the liquid 314 to the liquid container 313 through the liquid return tube 322 by pressure-feeding the liquid 314 in the liquid supply tube 316, and can supply the liquid 314 to the liquid droplet discharger 302 at any time. The liquid supply tube 316 and the drying collecting unit 360 are equipped with pressure gauges P1 and P2, respectively. The pressure gauges P1 and P2 monitor the liquid feed pressure toward the liquid droplet discharger 302 and the inner pressure of the drying collecting unit 360, respectively. When the pressure measured by the pressure gauge P1 is greater than that measured by the pressure gauge P2, there is a concern that the liquid 314 leaks from the discharge holes 19. When the pressure measured by the pressure gauge P1 is smaller than that measured by the pressure gauge P2, there is a concern that a gas flows in the liquid droplet discharger 302 and the liquid droplet discharge phenomenon is stopped. Thus, preferably, the pressure measured by the pressure gauge P1 is nearly identical to that measured by the pressure gauge P2.

Within a chamber 361, a descending conveyance gas stream 301 is formed through a conveyance gas stream inlet 364. Liquid droplets 321 discharged from the liquid droplet discharger 302 are conveyed downward by the action of gravity as well as the conveyance gas stream 301, and are collected by the particle collector 362 after passing through the conveyance gas stream discharge port 365 and stored in the particle storage portion 363.

In the liquid droplet discharge step, if the discharged liquid droplets come into contact with each other before being dried, the liquid droplets may coalesce with each other. In order to obtain particles having a narrow particle size distribution, it is preferable that the distance between the discharged liquid droplets is maintained. However, although having a constant initial velocity, the discharged liquid droplet rapidly stalls due to air resistance. When the liquid droplets discharged later catch up with the stalled liquid droplets and the drying of the liquid droplets is insufficient, the liquid droplets may coalesce with each other. To prevent coalescence, it is preferable that the liquid droplets are prevented from slowing down, that the conveyance gas stream 301 is used to convey the liquid droplets while being dried in a manner to prevent coalescence so as not to bring the liquid droplets into contact with each other. Therefore, the conveyance gas stream 301 is preferably arranged in the same direction as the liquid droplet discharge direction in the vicinity of the liquid droplet discharger 302. Note that even when the liquid droplets come into contact with each other, the liquid droplets will not coalesce if the liquid droplets are sufficiently dried by the time of contact. In such a case, the conveyance gas stream 301 does not necessarily have to be used.

FIG. 4 is an enlarged view of a liquid droplet discharger used in the particle production apparatus illustrated in FIG. 3. As illustrated in FIG. 4, the liquid droplet discharger 302 includes a volume changer 320, an elastic plate 309, and a liquid containing portion 319. When a voltage is applied to the volume changer 320, the liquid droplet discharger 302 is deformed to reduce the volume of the liquid containing portion 319, thereby discharging the liquid stored in the liquid containing portion 319 from the discharge hole as the liquid droplet 321.

FIG. 5 is a view illustrating another embodiment of the liquid droplet discharger of the particle production apparatus. As illustrated in FIG. 5, in the gas stream passage 312, the conveyance gas stream 301 may be in a direction substantially perpendicular to the discharge direction. Note that conveyance gas stream 301 can flow at a certain angle, preferably at such an angle that the liquid droplets are brought away from the liquid droplet discharger 302. As illustrated in FIG. 5, in the case where the liquid droplet 321 is discharged by changing the volume of the liquid containing portion 319 through the elastic plate 309 by the volume changer 320 and the coalescence preventing conveyance gas stream 301 is applied to the discharged liquid droplet 321 from the substantially perpendicular direction, it is preferable to arrange the discharge holes so that the loci of the liquid droplets 321 carried by the coalescence preventing conveyance gas stream 301 from the discharge holes do not overlap.

The particles may be conveyed to the particle collector by another gas stream after the coalescence is prevented by the conveyance gas stream 301.

The speed of the conveyance gas stream is preferably equal to or higher than the liquid droplet discharge speed. When the speed of the conveyance gas stream is higher than the liquid droplet discharge speed, coalescence of the liquid droplets can be prevented.

A chemical substance that promotes drying of the liquid droplets may be mixed into the conveyance gas stream. The state of the conveyance gas stream is not limited, and may be a laminar flow, a swirling flow, or a turbulent flow. The type of the gas constituting the conveyance gas stream is not particularly limited and may be appropriately selected according to the purpose. The conveyance gas stream may be constituted by air or a nonflammable gas such as nitrogen.

The temperature of the conveyance gas stream may be appropriately adjusted according to the purpose, but is a temperature at which the physiological activity of the physiologically active substance contained in the liquid droplet does not change due to the temperature of the gas flow.

When particles collected in the particle collector 362 illustrated in FIG. 3 contain a large amount of residual solvent, the particles are preferably subjected to a secondary drying to reduce the amount residual solvent if necessary. The secondary drying can be performed with a commonly known dryers such as a fluidized bed dryer and a vacuum dryer.

Rayleigh Fission Liquid Droplet Discharger

A discharger utilizing a Rayleigh fission method, as an example of the liquid droplet discharger, will be described with reference to FIG. 6.

FIG. 6 is a schematic cross-sectional view illustrating an example of the Rayleigh fission liquid droplet discharger. For example, as illustrated in FIG. 6, the liquid droplet discharger preferably includes at least a reservoir 401 for storing a raw material fluid, a vibrator 402, and a plurality of through holes 403.

Hereinafter, the Rayleigh fission liquid droplet discharger will be described in more detail for each member.

Reservoir

The reservoir needs to at least retain the raw material fluid in a pressurized state. Therefore, the reservoir is preferably made of a metal such as SUS or aluminum and has a pressure resistance of about 10 MPa, but is not limited thereto. Further, for example, as illustrated in FIG. 6, a structure in which a penetration holding mechanism 405 which is connected by a supply tube 404 for supplying the liquid to the reservoir and holds a plate having through holes is provided is desirable. The vibrator 402 that vibrates the entire reservoir is in contact with the reservoir. Preferably, the vibrator 402 is connected to a vibration generator 406 via a conductive wire 407 and controlled thereby. An open valve 408 that adjusts the pressure in the reservoir and removes bubbles therein is preferably provided for stable formation of liquid columns.

Vibrator

The vibrator 402 preferably vibrates the entire reservoir having the through holes by one vibrator. The vibrator 402 that vibrates the reservoir 401 is not particularly limited and may be appropriately selected and used as long as it can reliably vibrate at a constant frequency. From the above-described viewpoint, for example, the through holes are preferably vibrated at a constant frequency by expansion and contraction of the piezoelectric body.

The piezoelectric body has a function of converting electrical energy into mechanical energy. Specifically, expansion and contraction are caused by applying a voltage, and the through holes can be vibrated by the expansion and contraction.

The piezoelectric body may be made of a piezoelectric ceramic, such as lead zirconate titanate (PZT), which is generally laminated because of having a small displacement. Additionally, piezoelectric polymers such as polyvinylidene fluoride (PVDF), crystals, and single crystals of $LiNbO_3$, $LiTaO_3$, and $KNbO_3$ are also usable.

The constant frequency is not particularly limited and may be appropriately selected according to the purpose, but is preferably from 10kHz to 10 MHz, both inclusive, more preferably from 50kHz to 2 MHz, both inclusive, from the viewpoint of generating minute liquid droplets having an extremely uniform particle size.

Having generally described preferred embodiments of this disclosure, further understanding can be obtained by reference to certain specific examples which are provided herein for the purpose of illustration only and are not intended to be limiting. In the descriptions in the following examples, the numbers represent weight ratios in parts, unless otherwise specified.

EXAMPLES

Further understanding can be obtained by reference to certain Examples which are provided herein for the purpose of illustration only and are not intended to be limiting.

Example 1

Preparation of Prescription liquid A

Acetaminophen (available from Fujifilm Wako Pure Chemical Industries, Ltd.) as a physiologically active substance and a lactic acid-glycolic acid copolymer (PURASORB PDLG5004 (weight average molecular weight (Mw): 44,000), available from Covion Japan Co., Ltd.) as a base material were dissolved in a mixed solvent of acetonitrile as a solvent and water with a mass ratio of 90:10 to prepare a prescription liquid A. The prescription liquid A was prepared so that the content of acetaminophen was 0.2% by mass and the content of the lactic acid-glycolic acid copolymer was 3.8% by mass, relative to the total amount of the prescription liquid A.

Preparation of Particle 1 (Rayleigh Fission)

The Rayleigh fission liquid droplet discharger illustrated in FIG. 6 was used to discharge the obtained prescription liquid A from the discharge hole to form liquid droplets, and the particle production apparatus illustrated in FIG. 3 was used to remove the solvent from the liquid droplets to prepare particles 1. $T_d$ - $T_m$ calculated from the dry gas stream temperature, relative humidity, vapor pressure of the solvent, and the like, was 9.4 degrees Celsius, and $Y_m$-Y was 0.10.

The obtained particles 1 had a value of surface area/volume of 0.28, a number average particle size (Dn) of 14.8 $\mu$m, and an (R. S. F) of 0.58. These were measured using a particle size distribution analyzer by a laser diffraction scattering particle size distribution analyzer (device name: LA-960, available from HORIBA, Ltd.). Thereafter, the surface area/volume and the number average particle size (R. S. F) were measured in the same manner. The production conditions of the particles are as follows.

[Particle Production Conditions]

- Shape of discharge hole: True circle
- Diameter of discharge hole: 35 $\mu$m
- Pressure for extruding the prescription liquid: 0.18 MPa
- Excitation frequency: 80 kHz
- Excitation voltage: 5 V
- Conveyance gas stream amount: 50 $m^3$/h
- Temperature of dry gas stream: 26 degrees Celsius
- Relative humidity of the dry gas stream: 13%

Example 2

Preparation of Prescription liquid B

TRITC-labeled dextran (available from COSMO BIO COMPANY, LIMITED) as a physiologically active substance was dissolved in water to a concentration of 3% by mass to obtain a solution. Using a syringe (Syringe with Needle 25G available from Terumo Corporation), the solution was poured into a mixed solvent containing equal amounts of acetonitrile and dichloromethane to obtain a dispersion of TRITC-dextran. Lactic acid-glycolic acid copolymers (PURASORB PDLG5004 (weight average molecular weight (Mw): 44,000), available from Covion Japan Co., Ltd.) as a base material were dissolved in this dispersion to prepare a prescription liquid B. The prescription liquid B was prepared so that the content of TRITC-dextran was 0.04% by mass and the content of lactic acid-glycolic acid copolymer was 3.96% by mass relative to the total amount of the prescription liquid B.

Preparation of Particle 2 (Rayleigh Fission)

The Rayleigh fission liquid droplet discharger illustrated in FIG. 6 was used to discharge the obtained prescription liquid B from the discharge hole to form liquid droplets, and the particle production apparatus illustrated in FIG. 3 was used to remove the solvent from the liquid droplets to prepare particles 2. $T_d$ - $T_m$ calculated from the dry gas stream temperature, relative humidity, vapor pressure of the solvent, and the like, was 5.7 degrees Celsius, and $Y_m$-Y was 0.13.
The obtained particles 2 had a value of surface area/volume of 0.27, a number average particle size (Dn) of 16.5 $\mu$m, and an (R. S. F) of 0.56. The production conditions of the particles are as follows.

Particle Production Conditions

- Shape of discharge hole: True circle
- Diameter of discharge hole: 35 $\mu$m
- Pressure for extruding the prescription liquid: 0.18 MPa
- Excitation frequency: 80 kHz
- Excitation voltage: 5 V
- Conveyance gas stream amount: 50 m$^3$/h
- Temperature of dry gas stream: 60 degrees Celsius
- Relative humidity of the dry gas stream: 2.1%

Example 3

Preparation of Particle 3 (Rayleigh Fission)

Particles 3 were prepared in the same manner as in Example 2 except that the prescription liquid B was subjected to preparation under the following particle production conditions. $T_d$ - $T_m$ calculated from the dry gas stream temperature, relative humidity, vapor pressure of the solvent, and the like, was 18.3 degrees Celsius, and $Y_m$-Y was 0.11.
The obtained particles 3 had a value of surface area/volume of 0.27, a number average particle size (Dn) of 21.5 $\mu$m, and an (R. S. F) of 0.71. The production conditions of the particles are as follows.

[Particle Production Conditions]

- Shape of discharge hole: True circle
- Diameter of discharge hole: 35 $\mu$m
- Pressure for extruding the prescription liquid: 0.18 MPa
- Excitation frequency: 80 kHz
- Excitation voltage: 5 V
- Conveyance gas stream amount: 50 m$^3$/h
- Temperature of dry gas stream: 50 degrees Celsius
- Relative humidity of the dry gas stream: 7.3%

Example 4

Preparation of Prescription liquid C

Albumin (available from Fujifilm Wako Pure Chemical Industries, Ltd.) as a physiologically active substance and dextran (available from Fujifilm Wako Pure Chemical Industries, Ltd.) as a stabilizer were dissolved in water each to a concentration of 1.5% by mass to obtain a solution. Using a syringe (Syringe with Needle 25G available from Terumo Corporation), the solution was poured into a mixed solvent containing equal amounts of acetonitrile and dichloromethane to obtain a dispersion of albumin and dextran. Lactic acid-glycolic acid copolymers (PURASORB PDLG5004 (weight average molecular weight (Mw): 44,000), available from Covion Japan Co., Ltd.) as a base material were dissolved in this dispersion to prepare a prescription liquid C. The prescription liquid C was prepared so that the content of albumin and dextran was 0.02% by mass and the content of lactic acid-glycolic acid copolymer was 1.98% by mass relative to the total amount of the prescription liquid C.

Preparation of Particle 4 (Rayleigh Fission)

The Rayleigh fission liquid droplet discharger illustrated in FIG. 6 was used to discharge the obtained prescription liquid C from the discharge hole to form liquid droplets, and the particle production apparatus illustrated in FIG. 3 was used to remove the solvent from the liquid droplets to prepare particles 4. $T_d$ - $T_m$ calculated from the dry gas stream temperature, relative humidity, vapor pressure of the solvent, and the like, was 15.7 degrees Celsius, and $Y_m$-Y was 0.10.

The obtained particles 4 had a value of surface area/volume of 0.28, a number average particle size (Dn) of 17.2 $\mu$m, and an (R. S. F) of 0.76. The production conditions of the particles are as follows.

[Particle Production Conditions]

- Shape of discharge hole: True circle
- Diameter of discharge hole: 35 $\mu$m
- Pressure for extruding the prescription liquid: 0.18 MPa
- Excitation frequency: 80 kHz
- Excitation voltage: 5 V
- Conveyance gas stream amount: 50 m$^3$/h
- Temperature of dry gas stream: 40 degrees Celsius
- Relative humidity of the dry gas stream: 8.7%

Example 5

Preparation of Prescription liquid D

Ibuprofen (available from Tokyo Chemical Industry Co., Ltd.) as a physiologically active substance and Eudragit-RLPO (available from Evonik) as a base material were added to a mixed solvent containing equal amounts of acetonitrile and dichloromethane as a solvent to prepare a prescription liquid D. The prescription liquid D was prepared so that the ibuprofen content was 0.03% by mass and the Eudragit-RLPO content was 0.27% by mass relative to the total amount of the prescription liquid D.

Preparation of Particles 5 (Spray Drying)

Particles 5 were prepared by subjecting the obtained prescription liquid D to the following particle production conditions by using a spray drying unit (rotary disk atomizer, available from Ohkawara Kakohki Co., Ltd.). $T_d$ - $T_m$ calculated from the dry gas stream temperature, relative humidity, vapor pressure of the solvent, and the like, was 7.1 degrees Celsius, and $Y_m$-Y was 0.13.

The obtained particles 5 had a value of surface area/volume of 0.58, a number average particle size (Dn) of 11.5 $\mu$m, and an (R. S. F) of 1.0. The production conditions of the particles are as follows.

[Particle Production Conditions]

- Model of spray drying equipment: L-8
- Rotary disk atomizer rotational speed: 10, 000 rpm
- Feed per rotation of prescription liquid: 2 kg/time
- Conveyance gas stream amount: 50 m$^3$/h
- Temperature of dry gas stream: 60 degrees Celsius

- Relative humidity of the dry gas stream: 2.9%

Example 6

Preparation of Particle 2 (Rayleigh Fission)

Particles 6 were prepared in the same manner as in Example 2 except that the lactic acid-glycolic acid copolymers used in the prescription liquid B in Example 2 were changed to PURASORB PDLG5002 (weight average molecular weight (Mw): 17,000, available from Covion Japan Co., Ltd.), and the obtained prescription liquid was subjected to preparation under the following particle production conditions. $T_d$ - $T_m$ calculated from the dry gas stream temperature, relative humidity, vapor pressure of the solvent, and the like, was 14.5 degrees Celsius, and $Y_m$-Y was 0.13.
The obtained particles 6 had a value of surface area/volume of 0.27, a number average particle size (Dn) of 22.2 $\mu$m, and an (R. S. F) of 0.72. The production conditions of the particles are as follows.

[Particle Production Conditions]

- Shape of discharge hole: True circle
- Diameter of discharge hole: 35 $\mu$m
- Pressure for extruding the prescription liquid: 0.18 MPa
- Excitation frequency: 80 kHz
- Excitation voltage: 5 V
- Conveyance gas stream amount: 50 m$^3$/h
- Temperature of dry gas stream: 60 degrees Celsius
- Relative humidity of the dry gas stream: 4%

Example 7

Preparation of Particle 7 (Rayleigh Fission)

Particles 7 were prepared in the same manner as in Example 2, except that a mixed solvent having a composition of acetonitrile and dichloromethane at a ratio of 100:0 was used, and the obtained prescription liquid was subjected to preparation under the following particle production conditions. $T_d$ - $T_m$ calculated from the dry gas stream temperature, relative humidity, vapor pressure of the solvent, and the like, was 5.6 degrees Celsius, and $Y_m$-Y was 0.05.
The obtained particles 7 had a value of surface area/volume of 0.12, a number average particle size (Dn) of 19.7 $\mu$m, and an (R. S. F) of 0.81. The production conditions of the particles are as follows.

[Particle Production Conditions]

- Shape of discharge hole: True circle
- Diameter of discharge hole: 35 $\mu$m
- Pressure for extruding the prescription liquid: 0.18 MPa
- Excitation frequency: 80 kHz
- Excitation voltage: 5 V
- Conveyance gas stream amount: 50 m$^3$/h
- Temperature of dry gas stream: 40 degrees Celsius
- Relative humidity of the dry gas stream: 13.6%

Example 8

Preparation of Particle 8 (Rayleigh Fission)

Particles 8 were prepared in the same manner as in Example 2, except that a mixed solvent having a composition of acetonitrile and dichloromethane at a ratio of 75:25 was used, and the obtained prescription liquid was subjected to preparation under the following particle production conditions. $T_d$ - $T_m$ calculated from the dry gas stream temperature, relative humidity, vapor pressure of the solvent, and the like, was 5.6 degrees Celsius, and $Y_m$-Y was 0.07.

The obtained particles 8 had a value of surface area/volume of 0.16, a number average particle size (Dn) of 15.7 $\mu$m, and an (R. S. F) of 0.61. The production conditions of the particles are as follows.

[Particle Production Conditions]

- Shape of discharge hole: True circle
- Diameter of discharge hole: 35 $\mu$m
- Pressure for extruding the prescription liquid: 0.18 MPa
- Excitation frequency: 80 kHz
- Excitation voltage: 5 V
- Conveyance gas stream amount: 50 m$^3$/h
- Temperature of dry gas stream: 40 degrees Celsius
- Relative humidity of the dry gas stream: 6.4%

Example 9

Preparation of Particle 8 (Rayleigh Fission)

Particles 9 were prepared in the same manner as in Example 2 except that a mixed solvent having a composition of acetonitrile and dichloromethane at a ratio of 68:32 was used, and the obtained prescription liquid was subjected to preparation under the following particle production conditions. $T_d$ - $T_m$ calculated from the dry gas stream temperature, relative humidity, vapor pressure of the solvent, and the like, was 5.4 degrees Celsius, and $Y_m$-Y was 0.08.

The obtained particles 9 had a value of surface area/volume of 0.15, a number average particle size (Dn) of 16.6 $\mu$m, and an (R. S. F) of 0.57. The production conditions of the particles are as follows.

[Particle Production Conditions]

- Shape of discharge hole: True circle
- Diameter of discharge hole: 35 $\mu$m
- Pressure for extruding the prescription liquid: 0.18 MPa
- Excitation frequency: 80 kHz
- Excitation voltage: 5 V
- Conveyance gas stream amount: 50 m$^3$/h
- Temperature of dry gas stream: 40 degrees Celsius
- Relative humidity of the dry gas stream: 5.4%

Example 10

Preparation of Particle 10 (Rayleigh Fission)

Particles 10 were prepared in the same manner as in Example 2, except that a mixed solvent having a composition of acetonitrile and dichloromethane at a ratio of 62:38 was used, and the obtained prescription liquid was subjected to preparation under the following particle production conditions. $T_d$ - $T_m$ calculated from the dry gas stream temperature, relative humidity, vapor pressure of the solvent, and the like, was 5.3 degrees Celsius, and $Y_m$-Y was 0.09.

The obtained particles 10 had a value of surface area/volume of 0.14, a number average particle size (Dn) of 21.3 $\mu$m, and an (R. S. F) of 0.49. The production conditions of the particles are as follows.

[Particle Production Conditions]

- Shape of discharge hole: True circle
- Diameter of discharge hole: 35 $\mu$m

- Pressure for extruding the prescription liquid: 0.18 MPa
- Excitation frequency: 80 kHz
- Excitation voltage: 5 V
- Conveyance gas stream amount: 50 m$^3$/h
- Temperature of dry gas stream: 40 degrees Celsius
- Relative humidity of the dry gas stream: 4.8%

Comparative Example 1

Preparation of Particle 11 (Rayleigh Fission)

Particles 11 were prepared in the same manner as in Example 2 except that the obtained prescription liquid B was subjected to preparation under the following particle production conditions. $T_d$ - $T_m$ calculated from the dry gas stream temperature, relative humidity, vapor pressure of the solvent, and the like, was 26.4 degrees Celsius, and $Y_m$-Y was 0.09.
The obtained particles 11 had a value of surface area/volume of 0.26, a number average particle size (Dn) of 17.5 $\mu$m, and an (R. S. F) of 0.61. The production conditions of the particles are as follows.

[Particle Production Conditions]

- Shape of discharge hole: True circle
- Diameter of discharge hole: 35 $\mu$m
- Pressure for extruding the prescription liquid: 0.18 MPa
- Excitation frequency: 80 kHz
- Excitation voltage: 5 V
- Conveyance gas stream amount: 50 m$^3$/h
- Temperature of dry gas stream: 35 degrees Celsius
- Relative humidity of the dry gas stream: 22%

Comparative Example 2

Preparation of Particle 12 (Rayleigh Fission)

Particles 12 were prepared in the same manner as in Example 2, except that the obtained prescription liquid B was subjected to preparation under the following particle production conditions. $T_d$ - $T_m$ calculated from the dry gas stream temperature, relative humidity, vapor pressure of the solvent, and the like, was 27.9 degrees Celsius, and $Y_m$-Y was 0.08.
The obtained particles 12 had a value of surface area/volume of 0.28, a number average particle size (Dn) of 16.7 $\mu$m, and an (R. S. F) of 0.58. The production conditions of the particles are as follows.

[Particle Production Conditions]

- Shape of discharge hole: True circle
- Diameter of discharge hole: 35 $\mu$m
- Pressure for extruding the prescription liquid: 0.18 MPa
- Excitation frequency: 80 kHz
- Excitation voltage: 5 V
- Conveyance gas stream amount: 50 m$^3$/h
- Temperature of dry gas stream: 26 degrees Celsius
- Relative humidity of the dry gas stream: 35%

Comparative Example 3

Preparation of Particle 13 (Rayleigh Fission)

Particles 13 were prepared in the same manner as in Example 4, except that the obtained prescription liquid C was subjected to preparation under the following particle production conditions. $T_d - T_m$ calculated from the dry gas stream temperature, relative humidity, vapor pressure of the solvent, and the like, was 23.6 degrees Celsius, and $Y_m-Y$ was 0.10.

The obtained particles 13 had a value of surface area/volume of 0.26, a number average particle size (Dn) of 17.9 μm, and an (R. S. F) of 0.74. The production conditions of the particles are as follows.

[Particle Production Conditions]

- Shape of discharge hole: True circle
- Diameter of discharge hole: 35 μm
- Pressure for extruding the prescription liquid: 0.18 MPa
- Excitation frequency: 80 kHz
- Excitation voltage: 5 V
- Conveyance gas stream amount: 50 m$^3$/h
- Temperature of dry gas stream: 40 degrees Celsius
- Relative humidity of the dry gas stream: 15%

Next, the particles 1 to 13 obtained in Examples 1 to 10 and Comparative Examples 1 to 3 were used to measure and evaluate the "encapsulation rate (%) of physiologically active substance" (hereinafter occasionally referred to as "drug content rate (%)") and "initial burst (%)" were measured and evaluated as follows. The results are shown in Table 3.

[Case Where Physiologically Active Substance is Acetaminophen or Ibuprofen]

Measurement of Initial Burst (%)

The initial burst (%) of particles containing acetaminophen or ibuprofen as a physiologically active substance was measured by the following procedure.

A total of 20 mg of the particles and 30 mL of phosphate-buffered physiological saline (available from Fujifilm Wako Pure Chemical Industries, Ltd.) were placed in a 50 mL conical tube, and the conical tube was shaken (digital shaker, available from Front Lab) at 100 rpm.

After 10 minutes, 1 mL of the liquid in the conical tube was collected and centrifuged, and 0.5 mL of the supernatant was placed in a 2 mL vial. Further, 0.5 mL of aqueous acetonitrile (acetonitrile: water = 9: 1) was added to the 2 mL vial.

Using this solution, the amount of the physiologically active substance (acetaminophen or ibuprofen) eluted was measured by liquid chromatography under the following conditions. The "initial burst (%)" was calculated based on the measured elution amount and the following formula. The results are shown in Table 3.

[Conditions for Liquid Chromatography]

- Column: XBridge C18 3.5 μm (Waters) (Particle size: 3.5 μm, column size:4.6 × 150mm)
- Column temperature: 80 degrees Celsius
- Mobile phases: (A) pure water; (B) acetonitrile, A:B = 10:90
- Injection volume: 10 μL
- Detector: UV-VIS detector (Agilent Technologies); 210 nm
- Flow rate: 1.0 mL/min

[Initial Burst (%) Calculation Formula]

$$\text{Initial burst (\%) = (amount of physiologically active substance in test liquid/amount}$$

$$\text{of physiologically active substance present in prepared particles)} \times 100 \, (\%)$$

Measurement of Encapsulation Rate (%)

In order to measure the ratio of the physiologically active substance (acetaminophen or ibuprofen) in the prepared particles, 2 mg of each of the particles was weighed and dissolved in 10 mL of the above-mentioned aqueous acetonitrile (acetonitrile: water = 9: 1). Using this solution, the physiologically active substance was quantitatively evaluated by liquid chromatography. The encapsulation rate (%) was calculated using the following formula. The results are shown in Table 3. The conditions for the liquid chromatography are the same as those in the measurement of the initial burst (%) when the physiologically active substance is acetaminophen or ibuprofen.

[Encapsulation Rate (%) Calculation Formula]

$$\text{Encapsulation rate (\%) = (amount of physiologically active substance present in}$$

$$\text{prepared particles / charged amount of physiologically active substance)} \times 100 \, (\%)$$

[Case Where Physiologically Active Substance is TRITC-Labeled Dextran]

Measurement of Initial Burst (%)

The initial burst (%) of particles containing TRITC-labeled dextran as a physiologically active substance was measured by the following procedure.

A total of 10 mg of the particles and 500 $\mu$L of phosphate-buffered physiological saline (available from Fujifilm Wako Pure Chemical Industries, Ltd.) were placed in a 1.5 mL micro-tube, and the micro-tube was shaken (digital shaker, available from Front Lab) at 100 rpm. After 10 minutes, 400 $\mu$L of the liquid in the micro-tube was collected and centrifuged, and 200 $\mu$L of the supernatant was placed in a 2 mL vial.

Using this solution, the amount of the physiologically active substance (TRITC-labeled dextran) eluted was measured by a multi-label reader under the following conditions. The "initial burst (%)" was calculated based on the measured elution amount and the following formula. The results are shown in Table 3.

[Conditions of Multi-Label Reader]

- Model: ARVO X4 available from Perkin-Elmer Co., Ltd.
- Measurement type: Fluorescence intensity measurement
- Excitation filter: 544/15 nm
- Detection filter: 579/25 nm

[Initial Burst (%) Calculation Formula]

$$\text{Initial burst (\%) = (amount of physiologically active substance in test liquid/amount}$$

$$\text{of physiologically active substance present in prepared particles)} \times 100 \, (\%)$$

Measurement of Encapsulation Rate (%)

In order to measure the ratio of the physiologically active substance (TRITC-labeled dextran) in the prepared particles, 5 mg of the particles was weighed and dissolved in 1 mL of a 10% by mass aqueous sodium hydroxide. Using this solution, the physiologically active substance was quantitatively evaluated by a multi-label reader. The encapsulation rate (%) was calculated using the following formula. The results are shown in Table 3. The conditions for the multi-label reader are the same as those in the measurement of the initial burst (%) when the physiologically active substance is TRITC-labeled dextran.

[Encapsulation Rate (%) Calculation Formula]

$$\text{Encapsulation rate } (\%) = (\text{amount of physiologically active substance present in prepared particles / charged amount of physiologically active substance}) \times 100 \ (\%)$$

[Case Where Physiologically Active Substance is Albumin]

Measurement of Initial Burst (%)

The initial burst (%) of particles containing albumin as a physiologically active substance was measured by the following procedure.

A total of 10 mg of the particles and 500 μL of phosphate-buffered physiological saline (available from Fujifilm Wako Pure Chemical Industries, Ltd.) were placed in a 1.5 mL micro-tube, and the micro-tube was shaken (digital shaker, available from Front Lab) at 100 rpm. After 10 minutes, 400 μL of the liquid in the micro-tube was collected and centrifuged, and 200 μL of the supernatant was placed in a 2 mL vial.

Using this solution, the amount of the physiologically active substance (albumin) eluted was measured by a multi-label reader under the following conditions. The "initial burst (%)" was calculated based on the measured elution amount and the following formula. The results are shown in Table 3.

[Conditions of Multi-Label Reader]

- Model: ARVO X4 available from Perkin-Elmer Co., Ltd.
- Measurement type: Ultraviolet absorbance measurement 280 nm

[Initial Burst (%) Calculation Formula]

$$\text{Initial burst } (\%) = (\text{amount of physiologically active substance in test liquid/amount of physiologically active substance present in prepared particles}) \times 100 \ (\%)$$

Measurement of Encapsulation Rate (%)

In order to measure the ratio of the physiologically active substance (albumin) in the prepared particles, 5 mg of the particles was weighed and dissolved in 1 mL of a 10% by mass aqueous sodium hydroxide. Using this solution, the physiologically active substance was quantitatively evaluated by a multi-label reader. The encapsulation rate (%) was calculated using the following formula. The results are shown in Table 3. The conditions for the multi-label reader are the same as those in the measurement of the initial burst (%) when the physiologically active substance is albumin.

[Encapsulation Rate (%) Calculation Formula]

$$\text{Encapsulation rate (\%)} = (\text{amount of physiologically active substance present in}$$

$$\text{prepared particles / charged amount of physiologically active substance)} \times 100 \text{ (\%)}$$

The above are summarized in the following Tables 1 to 3. In Table 1, "PLGA-1" represents "lactic acid-glycolic acid copolymers (PURASORB PDLG5004 (weight average molecular weight (Mw): 44,000))", and "PLGA-2" represents "lactic acid-glycolic acid copolymers (PURASORB PDLG5002 (weight average molecular weight (Mw): 17,000))".

Table 1

| | | Prescription liquid | | | | | |
| | | Physiologically active substance/ stabilizer | | | Base material | | Solvent |
| | | Type | Content (%by mass) | State in the solution | Type | Content (%by mass) | Type |
| Examples | 1 | Acetaminophen | 0.20 | Dissolved | PLGA-1 | 3.80 | Acetonitrile: water = 90:10 |
| | 2 | TRITC-labeled dextran | 0.04 | Dispersed | PLGA-1 | 3.96 | Acetonitrile: dichloromethane = 50:50 |
| | 3 | TRITC-labeled dextran | 0.04 | Dispersed | PLGA-1 | 3.96 | Acetonitrile: dichloromethane = 50:50 |
| | 4 | Albumin/dextran | 0.02 | Dispersed | PLGA-1 | 1.98 | Acetonitrile: dichloromethane = 50:50 |
| | 5 | Ibuprofen | 0.03 | Dissolved | Eudragit -RLPO | 0.27 | Acetonitrile: dichloromethane = 50:50 |
| | 6 | TRITC-labeled dextran | 0.04 | Dispersed | PLGA-2 | 3.96 | Acetonitrile: dichloromethane = 50:50 |
| | 7 | TRITC-labeled dextran | 0.04 | Dispersed | PLGA-1 | 3.96 | Acetonitrile: dichloromethane = 100:0 |
| | 8 | TRITC-labeled dextran | 0.04 | Dispersed | PLGA-1 | 3.96 | Acetonitrile: dichloromethane = 75:25 |
| | 9 | TRITC-labeled dextran | 0.04 | Dispersed | PLGA-1 | 3.96 | Acetonitrile: dichloromethane = 68:32 |
| | 10 | TRITC-labeled dextran | 0.04 | Dispersed | PLGA-1 | 3.96 | Acetonitrile: dichloromethane = 62:38 |

(continued)

| | | Prescription liquid | | | | | |
|---|---|---|---|---|---|---|---|
| | | Physiologically active substance/ stabilizer | | | Base material | | Solvent |
| | | Type | Content (%by mass) | State in the solution | Type | Content (%by mass) | Type |
| Comparative examples | 1 | TRITC-labeled dextran | 0.04 | Dispersed | PLGA-1 | 3.96 | Acetonitrile: dichloromethane = 50:50 |
| | 2 | TRITC-labeled dextran | 0.04 | Dispersed | PLGA-1 | 3.96 | Acetonitrile: dichloromethane = 50:50 |
| | 3 | Albumin/dextran | 0.02 | Dispersed | PLGA-1 | 1.98 | Acetonitrile: dichloromethane = 50:50 |

Table 2

| | | Method of producing particles | Drying condition | | | |
|---|---|---|---|---|---|---|
| | | | Gas stream temperature (degrees Celsius) | Relative humidity of gas streams (%) | $Y_m-Y$ | $T_d - T_m$ (degrees Celsius) |
| Examples | 1 | Rayleigh fission | 26 | 13 | 0.10 | 9.4 |
| | 2 | Rayleigh fission | 60 | 2.1 | 0.13 | 5.7 |
| | 3 | Rayleigh fission | 50 | 7.3 | 0.11 | 18.3 |
| | 4 | Rayleigh fission | 40 | 8.7 | 0.10 | 15.7 |
| | 5 | Spray drying | 60 | 2.9 | 0.13 | 7.1 |
| | 6 | Rayleigh fission | 60 | 4 | 0.13 | 14.5 |
| | 7 | Rayleigh fission | 40 | 13.6 | 0.05 | 5.6 |
| | 8 | Rayleigh fission | 40 | 6.4 | 0.07 | 5.6 |
| | 9 | Rayleigh fission | 40 | 5.4 | 0.08 | 5.4 |
| | 10 | Rayleigh fission | 40 | 4.8 | 0.09 | 5.3 |
| Comparative examples | 1 | Rayleigh fission | 35 | 22 | 0.09 | 26.4 |
| | 2 | Rayleigh fission | 26 | 35 | 0.08 | 27.9 |
| | 3 | Rayleigh fission | 40 | 15 | 0.10 | 23.6 |

Table 3

| | | Particles | | | Evaluation result | |
|---|---|---|---|---|---|---|
| | | Surface areal volume | Number average particle size Dn ($\mu$m) | R.S.F. | Drug content rate (%) | Initial burst (%) |
| Examples | 1 | 0.28 | 14.8 | 0.58 | 98 | 21 |
| | 2 | 0.27 | 16.5 | 0.56 | 99 | 6.8 |
| | 3 | 0.27 | 21.5 | 0.71 | 99 | 40.1 |
| | 4 | 0.28 | 17.2 | 0.76 | 95 | 35.1 |
| | 5 | 0.58 | 11.5 | 1.0 | 97 | 7.2 |
| | 6 | 0.27 | 22.2 | 0.72 | 99 | 44.1 |
| | 7 | 0.12 | 19.7 | 0.81 | 80 | 59.4 |
| | 8 | 0.16 | 15.7 | 0.61 | 90 | 52.7 |
| | 9 | 0.15 | 16.6 | 0.57 | 91 | 41.0 |
| | 10 | 0.14 | 21.3 | 0.49 | 90 | 9.2 |
| Comparative examples | 1 | 0.26 | 17.5 | 0.61 | 78 | 70.4 |
| | 2 | 0.28 | 16.7 | 0.58 | 76 | 76.5 |
| | 3 | 0.26 | 17.9 | 0.74 | 95 | 63.7 |

As illustrated in Tables 2 and 3, the measurement results of the initial burst (%) demonstrate that the initial burst (%) of the particles of Examples having a small value of $T_d$ - $T_m$ was lower than the initial burst (%) of the particles of Comparative Examples.

Therefore, it can be said that the initial burst is prevented in the particles of Examples and particles of Examples are suitable for a sustained releasing preparation.

Aspects according to the present disclosure include, for example, the following.

Aspect 1 A sustained releasing particle contains a water-soluble physiologically active substance and a fat-soluble base material, wherein the sustained releasing particle has a ratio of a surface area to a volume is 0.6 or less.

Aspect 2 The sustained releasing particle according to Aspect 1 having a number average particle size of from 1 to 50 $\mu$m and a relative span factor (R.S.F.) of 1.0 or less.

Aspect 3 The sustained releasing particle according to Aspect 1 or 2, wherein the fat-soluble base material contains at least one of polylactic acid or a lactic acid-glycolic acid copolymer.

Aspect 4 The sustained releasing particles according to Aspect 3, wherein the polylactic acid and the lactic acid-glycolic acid copolymer each have a weight average molecular weight of 40,000 or more.

Aspect 5 A method of producing a sustained releasing particle includes discharging a liquid droplet containing a physiologically active substance, a base material, and a solvent and removing the solvent from the liquid droplet by a dry gas stream on condition that the difference ($T_d$ - $T_m$) between the dew point temperature ($T_d$) of the dry gas stream and the wet bulb temperature ($T_m$) of the liquid droplet discharged is 20 degrees Celsius or less.

Aspect 6 The method according to Aspect 5, wherein, in the removing, the solvent is removed from the liquid droplet on condition that a difference ($Y_m$ - Y) is 0.07 or more, where $Y_m$ represents a humidity when the solvent is saturated in the liquid droplet at a wet bulb temperature of the dry gas stream and Y represents a humidity of the dry gas stream.

Aspect 7 The method according to Aspect 5 or 6, wherein the physiologically active substance is water-soluble.

Aspect 8 The method according to Aspect 5 or 6, wherein the physiologically active substance is dispersed in the solvent.

Aspect 9 The method according to Aspect 5 or 6, wherein the base material contains at least one of polylactic acid or a lactic acid-glycolic acid copolymer.

Aspect 10 The method according to Aspect 9, wherein the polylactic acid and the lactic acid-glycolic acid copolymer each have a weight average molecular weight of 40,000 or more.

The sustained releasing particles according to any one of Aspect 1 to Aspect 4 and the production method of sustained releasing particles according to any one of Aspect 5 to Aspect 10 can solve the conventional problems and achieve an objective according to the present disclosure.

The above-described embodiments are illustrative and do not limit the present invention. Thus, numerous additional modifications and variations are possible in light of the above teachings. For example, elements and/or features of different illustrative embodiments may be combined with each other and/or substituted for each other within the scope of the present invention.

**Claims**

1. A sustained releasing particle comprising:

   a water-soluble physiologically active substance; and
   a fat-soluble base material,
   wherein the sustained releasing particle has a ratio of a surface area to a volume is 0.6 or less.

2. The sustained releasing particle according to claim 1 having:

   a number average particle size of from 1 to 50 $\mu$m; and
   a relative span factor (R.S.F.) of 1.0 or less.

3. The sustained releasing particle according to claim 1 or 2,
   wherein the fat-soluble base material contains at least one of polylactic acid or a lactic-acid·glycolic acid copolymer.

4. The sustained releasing particle according to claim 3,
   wherein the polylactic acid and the lactic acid-glycolic acid copolymer each have a weight average molecular weight of 40,000 or more.

5. A method of producing a sustained releasing particle, comprising:

   discharging a liquid droplet containing a physiologically active substance, a base material, and a solvent; and
   removing the solvent from the liquid droplet by a dry gas stream on condition that a difference ($T_d$ - $T_m$) between a dew point temperature ($T_d$) of the dry gas stream and a wet bulb temperature ($T_m$) of the liquid droplet discharged is 20 degrees Celsius or less.

6. The method according to claim 5,
   wherein, in the removing, the solvent is removed from the liquid droplet on condition that a difference ($Y_m$ - $Y$) is 0.07 or more, where $Y_m$ represents a humidity when the solvent is saturated in the liquid droplet at a wet bulb temperature of the dry gas stream and $Y$ represents a humidity of the dry gas stream.

7. The method according to claim 5 or 6, wherein the physiologically active substance is water-soluble.

8. The method according to claim 5 or 6, wherein the physiologically active substance is dispersed in the solvent.

9. The method according to claim 5 or 6, wherein the base material contains at least one of polylactic acid or a lactic acid-glycolic acid copolymer.

10. The method according to claim 9, wherein the polylactic acid and the lactic acid-glycolic acid copolymer each have a weight average molecular weight of 40,000 or more.

# FIG. 1

VOLUME (%)

PARTICLE SIZE (μm)

══════ PARTICLE MANUFACTURED BY METHOD OF EMBODIMENT
── ── ── PARTICLE MANUFACTURED BY SPRAY DRYING METHOD
─ ─ ─ ─ PARTICLE MANUFACTURED BY SPRAY DRYING METHOD

# FIG. 2

# FIG. 3

# FIG. 4

# FIG. 5

# FIG. 6

Europäisches
Patentamt

European
Patent Office

Office européen
des brevets

# EUROPEAN SEARCH REPORT

Application Number

EP 23 19 6274

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/085314 A1 (MORINAGA TADAHIKO [JP] ET AL) 29 March 2018 (2018-03-29)<br>* figure 1; examples 1, 5 *<br>* paragraphs [0023], [0024], [0046] – [0083] *<br>* paragraphs [0087] – [0092] * | 1-10 | INV.<br>A61K9/16 |
| Y | EP 3 546 062 A1 (RICOH CO LTD [JP]) 2 October 2019 (2019-10-02)<br>* claim 2; examples 3, 5, 6, 7 *<br>* paragraphs [0027] – [0032] *<br>* paragraphs [0038] – [0047] *<br>* paragraphs [0056] – [0064] * | 1-10 | |
| Y | BOEL ELINE ET AL: "Unraveling Particle Formation: From Single Droplet Drying to Spray Drying and Electrospraying", PHARMACEUTICS,<br>vol. 12, no. 7, 4 July 2020 (2020-07-04), page 625, XP055962559,<br>DOI: 10.3390/pharmaceutics12070625<br>*"2.4.2. The Characteristic Drying Rate Curve (CDRC) Approach" on pages 22-24*;<br>figure 6<br>*"2.2.1. Drying Conditions" on pages 9-12*;<br>figure 4 | 1-10 | |

TECHNICAL FIELDS
SEARCHED      (IPC)

A61K

The present search report has been drawn up for all claims

| Place of search | Date of completion of the search | Examiner |
|---|---|---|
| Munich | 7 December 2023 | Gerber, Myriam |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 19 6274

This annex lists the patent family members relating to the patent documents cited in the above-mentioned European search report.
The members are as contained in the European Patent Office EDP file on
The European Patent Office is in no way liable for these particulars which are merely given for the purpose of information.

07-12-2023

| Patent document cited in search report | | Publication date | Patent family member(s) | | Publication date |
|---|---|---|---|---|---|
| US 2018085314 | A1 | 29-03-2018 | US 2018085314 | A1 | 29-03-2018 |
| | | | US 2022062178 | A1 | 03-03-2022 |
| EP 3546062 | A1 | 02-10-2019 | CN 110358116 | A | 22-10-2019 |
| | | | EP 3546062 | A1 | 02-10-2019 |
| | | | JP 7092974 | B2 | 29-06-2022 |
| | | | JP 2019167506 | A | 03-10-2019 |
| | | | US 2019292333 | A1 | 26-09-2019 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008047863 A
- JP 2021147330 A